(19) Europäisches Patentamt
European Patent Office
Office européen des brevets

(11) Publication number: **0 276 302 B1**

# (12) EUROPEAN PATENT SPECIFICATION

(45) Date of publication of patent specification: **28.04.93**

(51) Int. Cl.[5]: **C12Q 1/68**

(21) Application number: **87905826.1**

(22) Date of filing: **11.08.87**

(86) International application number:
**PCT/US87/01966**

(87) International publication number:
**WO 88/01302 (25.02.88 88/05)**

## (54) NUCLEIC ACID PROBE ASSAY METHODS AND COMPOSITIONS.

(30) Priority: **11.08.86 US 895756**

(43) Date of publication of application:
**03.08.88 Bulletin 88/31**

(45) Publication of the grant of the patent:
**28.04.93 Bulletin 93/17**

(84) Designated Contracting States:
**AT BE CH DE FR GB IT LI LU NL SE**

(56) References cited:
**EP-A- 0 070 687** **EP-A- 0 130 515**
**US-A- 2 277** **US-A- 4 302 204**
**US-A- 4 358 535** **US-A- 4 419 444**
**US-A- 4 469 796** **US-A- 4 486 539**

**NATURE, vol. 313, 24 January 1985, London (GB); L.RATNER et al., pp. 277-284**

**DNA, vol. 4, no. 4, August 1985, New York, NY (US); B.C.F.CHU et al., pp. 327-331**

(73) Proprietor: **SISKA DIAGNOSTICS,INC.**
**505 South Coast Boulevard**
**La Jolla, CA 92037(US)**

(72) Inventor: **GINGERAS, Thomas, Raymond**
**1528 Juniper Hill Drive**
**Encinitas, CA 92024(US)**
Inventor: **GHOSH, Soumitra, Shankar**
**9501 Genesee Avenue, F-2**
**San Diego, CA 92121(US)**
Inventor: **DAVIS, Geneva, Ruth**
**3083 East Fox Run Way**
**San Diego, CA 92111(US)**
Inventor: **KWOH, Dobrah, Yantis**
**2404 Jacaranda Avenue**
**Carlsbad, CA 92008(US)**
Inventor: **MUSSO, Gary, Fred**
**1454 Vanessa Circle**
**Encinitas, CA 92024(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.5x/3.0.1)

JOURNAL OF HISTOCHEMISTRY AND CYTOCHEMISTRY, vol. 29, no. 2, February 1981, New York, NY (US); J.G.J.BAUMAN et al., p. 227

NUCLEIC ACIDS RESEARCH, vol. 12, no. 8, 25 April 1984, Oxford, England (GB); M.RENZ et al., pp. 3435, 3441

JOURNAL OF BIOLOGICAL CHEMISTRY, vol. 250, no. 3, 10 February 1975, Baltimore, MD (US); T.PFEUFFER et al., p. 869

VIROLOGY, vol. 139, December 1984, New York, NY (US); S.F.JOSEPHS et al., p. 340

NATURE, vol. 317, 03 October 1985, London, England (GB); F.WONG-STAAL et al., pp. 395, 398

(74) Representative: **Baverstock, Michael George Douglas et al**
**BOULT, WADE & TENNANT 27 Furnival Street London, EC4A 1PO (GB)**

## Description

### TECHNICAL FIELD

The present invention relates to nucleic acid probe assay methods and compositions for use therein.

More particularly, the invention relates to such methods wherein target nucleic acid analyte in a sample being assayed is captured from solution by hybridization to a first probe, which is bound to a solid support. Among these methods are sandwich assay methods, wherein the target is detected by means of a second probe, which is labeled for detection and which is hybridized to a segment of target analyte other than the segment hybridized to first probe.

In the assay methods of concern for the present invention, the solid-support-bound probes are oligonucleotides with between about 15 and about 100 bases.

### BACKGROUND OF THE INVENTION

Nucleic acid probe assays for testing a sample for the presence of a particular nucleic acid analyte, and the application of such assays for diagnosing diseases of animals and plants, testing blood, food and other products for viral or microbial contamination, isolating, characterizing and engineering of genes and microorganisms, and other purposes are well known. See, e.g., Meinkoth and Wahl, Anal. Biochem. 138, 267-284 (1984); Maniatis et al., Molecular Cloning: A Laboratory Manual, Cold Spring Harbor Laboratory Press, Cold Spring Harbor, New York (1982); Owens and Diener, U.S. Patent No. 4,480,040; and Falkow and Moseley, U.S. Patent No. 4,358,535.

Among nucleic acid probe assays are "sandwich assays," described above. It has been found that, in a sandwich assay, the capture step can be carried out before the labeling step or capture and labeling can be carried out simultaneously. See, e.g. Langdale and Malcolm, Gene 36, 201-210 (1985); Ranki et al., Gene 21, 77-85 (1983); Dunn and Hassell, Cell 12, 23-36 (1977); Ranki and Soderlund, U.S. Patent Nos. 4,486,539 and 4,563,419.

Sandwich assay methods have the advantage that nucleic acid of a sample being assayed need not be immobilized directly to a solid support. Instead, capture probe is immobilized to solid support such as described in US-A-4486539, and this can be accomplished by a supplier rather than the party actually carrying out the assay. Sandwich assays, because they involve two hybridizations to analyte, also offer the promise of higher specificity than non-sandwich assays, which involve hybridization of a single, labeled probe to analyte.

The use of short oligonucleotides, with fewer than about 100 nucleotides, as probes in nucleic acid probe assays also offers certain advantages. The time required for hybridization between probe and analyte is reduced with short probes. With current technology for chemical synthesis and purification, single-stranded oligonucleotides of up to about 100 nucleotides in a defined sequence can be prepared in highly pure form in sufficient quantity and with sufficient speed for routine use in assays. Such preparations are simpler, yield probe of higher quality for purposes of sensitivity and specificity in nucleic acid probe assays, and are most cost-effective than preparations of longer probes, which, to be cost-effective, must employ biological processes (e.g. cloning).

A Sandwich assay method, in which the solid phase-bound "capture probe" has fewer than about 200 bases, is described in EP-A-0070687.

Various methods have been described for affixing single-stranded nucleic acids to solid supports, for affinity chromatography, wherein hybridization between nucleic acids with complementary sequences effects separation, and for nucleic acid probe assays, including sandwich assays. See, e.g. Langdale and Malcolm, supra; Bunemann and Westhoff, Meth. Enzym. 100, 400-407 (1983); Brunemann, Nucl. Acids Res. 10, 7181-7196 (1982); Brunemann et al., Nucl. Acids Res. 10, 7163-7180 (1982); Clerici et al., Nucl. Acids Res. 6,247-258 (1979). In US-A-4419444 the attachment of organic compounds in general to solid supports via the hydroxyl oxygen of a carbohydrate moiety is described. However, the attachment of nucleic acids to amino-derivatized and carboxyl-derivatized solid supports, particularly such attachment wherein at least about 30% of the nucleic acid probe preparation is attached covalently only through a hydroxyl oxygen of a terminal deoxyribose or ribose moiety, has not been described.

The present invention is directed to realizing the advantages of both sandwich assays and the use of short oligonucleotides, with fewer than about 100 bases, as probes in the assays.

SUMMARY OF THE INVENTION

Solid supports have been discovered which are especially well suited for use in nucleic acid probe sandwich assays, in which an oligonucleotide with 15-100 bases is employed to capture nucleic acid analyte from a hybridization solution. The solid supports of the invention are characterized by a high fraction (typically at least about 30%) of bound oligonucleotide "capture probe" being covalently bound to the support only through a linker joined covalently to the support and to the 5'-oxygen of the 5'-terminal nucleoside or the 3'-oxygen of the 3'-terminal nucleoside of the oligonucleotide, and thereby being fully available for capture of analyte, rather than being associated with the support in a manner whereby availability for analyte capture is significantly reduced or even eliminated, such as by non-covalent association or covalent binding through a reactive base moiety.

The solid supports of the invention are (1) macroporous cross-linked dextran, porous silicate glass, polystyrene, or latex derivatized with an amino-terminated linker, with terminal amino groups of linker moieties covalently joined through a phosphoramidate group to the terminal nucleoside of the oligonucleotide capture probe and with substantially all of the amino groups that are not covalently joined through a phosphoramidate group to the terminal nucleoside of the oligonucleotide capture probe being blocked with an acyl group from interacting non-specifically with the oligonucleotide; (2) macroporous cross-linked dextran which, after activation with cyanogen bromide, reacted with the amino group of the aminoalkylphosphoramidate-group derivatized to a terminal nucleoside of an oligonucleotide capture probe to covalently derivatize the cross-linked dextran with the probe; (3) macroporous cross-linked dextran, porous silicate glass, polystyrene or latex derivatized with a carboxyl-terminated or active-ester-terminated linker, with a fraction of the carboxyl groups joined in an amide linkage to one amino group of a linking diaminoalkyl or diaminoalkyl disulfide (such as cystaminyl) moiety, the other amino group of which is part of a phosphoramidate which, in turn, is bonded directly to the terminal nucleoside of the oligonucleotide capture probe; and (4) cross-linked dextran, porous silicate glass, latex or polystyrene derivatized with a sulphydryl-terminated linker, with a fraction of the sulhydryl groups joined in a disulfide linkage to a sulfur from a linker which has an activated disulfide (activated with,e.g., 2-pyridyl or 4-pyridyl) at one end and an amino group at the other, said amino group forming part of a phosphoramidate which is bonded directly to a terminal nucleoside of the probe, said disulfide linkage arising from disulfide exchange between the sulfhydryl group derivatized to the dextran, silicate, latex, or polystyrene and the activated disulfide of the linker bonded to the probe.

The preferred support materials, with which the solid supports according to the invention are made, are long chain alkylamine-derivatized and carboxyl-derivatized controlled pore glass as sold by Pierce Chemical Co., Rockford, Illinois, USA, under product numbers 24875 and 23735, respectively, in the form of beads with nominal pore diameter of 0.05$\mu$m (500 Angstroms) and particle diameters between about 125 and 177$\mu$m (microns), and the macroporous cross-linked dextran material Sephacryl® S-500, sold by Pharmacia Inc., Piscataway, New Jersey, USA under Code No. 17-0475-01 in the form of beads with a wet diameter of about 40 to about 105$\mu$m (microns) and an exclusion volume for dextrans of molecular weight above about $2 \times 10^7$ daltons, said Sephacryl® modified as described below to be derivatized with cyanogen bromide or with an amino-terminated, carboxyl-terminated, or active ester-terminated linker.

Reactions of the preferred support materials with the preferred capture probes, which are probes modified to have a suitable linking moiety bonded to their 5'-termini, are described below.

Novel and advantageous sandwich assay methods of the invention are based on discoveries of various unexpected properties, of solid supports according to the invention, relating to the performance of the supports in sandwich assays carried out in various ways.

The prior art suggests that prehybridizing a solid support (e.g., nitrocellulose filter), to which a target nucleic acid analyte has been bound, with a non-homologous (sometimes referred to herein as "inert") nucleic acid (such as sheared salmon sperm or herring sperm DNA, or boiled tRNA, which is not labeled for detection and which has little sequence homology to target nucleic acid analyte), that is in a solution to be incubated under hybridizing conditions with the solid support for the purpose of hybridizing to target nucleic acid on the support probe nucleic acid in the solution, will reduce non-specific binding of probe and, thereby, reduce background signal. See, e.g., Maniatis et al., supra and US-A-4302204. There is no suggestion in the prior art that prehybridization of solid support, derivatized with an oligonucleotide, with non-homologous nucleic acid will increase the efficiency of capture by the oligonucleotide during hybridization of nucleic acid having complementary sequence. We have found, entirely unexpectedly, that prehybridization of a solid-support according to the invention with a non-homologous nucleic acid nearly doubles the efficiency of capture of target analyte during hybridization with a solution which includes the target.

Further, the prior art suggests that the inclusion of dextran sulfate in hybridization solutions in which a short (less than 100 nucleotide) oligonucleotide is present will have no effect on the efficiency with which hybridization occurs between the oligonucleotide and nucleic acid with sequence complementary to that of the oligonucleotide. Unexpectedly to the contrary, we have discovered that the capture efficiency of oligonucleotides attached to solid supports of the invention is markedly improved with between about 5% (w/v) and 20% (w/v), preferably about 9% (w/v) to about 11% (w/v), dextran sulfate in the hybridization solution when the analyte is long, i.e., more than about 500 bases. We have also found that dextran sulfate in this concentration range in a prehybridization solution, even to the exclusion of non-homologous DNA, also enhances capture efficiency. However, we have found that, with significantly shorter analyte (about 30 bases), dextran sulfate has no effect on capture efficiency. Further, we have found that the effect that dextran sulfate has on capture efficiency with long analyte is not, as the prior art would suppose, a volume-exclusion effect, as polyethylene glycol has a very much smaller effect on capture efficiency. The basis for the advantageous effect we have found for dextran sulfate on capture efficiency by solid supports of the invention remains unclear, although it may be charge-related.

We have also discovered unexpectedly that results (i.e. intensity of signal, corrected for background, from labeled probe hybridized to analyte that is bead-bound through capture probe) obtained with a sandwich assay in which capture of analyte and hybridization of labeled probe with analyte occur simultaneously, are essentially the same as a sandwich assay in which analyte is first hybridized to a significant extent with labeled probe in solution and then capture of analyte, wholly or partially complexed with labeled probe, is carried out. The results obtained when the capture step is carried out before the hybridization step with labeled probe are significantly poorer than with the other two procedures, presumably because bead-bound analyte is less accessible to binding by labeled probe in solution.

Finally, a number of oligonucleotides, listed in the following Table I, have been identified, from the sequence provided by Ratner et al., Nature 313, 277-284 (1985), as suitable for use as probes in nucleic acid probe hybridization assays, including assays according to the present invention, for HIV-1, the causative agent of AIDS. In particular, the oligonucleotides have been found to hybridize, under stringency conditions comparable to those employed in assays according to the present invention, to HIV-1 genome (an RNA which integrates as a proviral DNA into the genome of infected cells) or a fragment thereof, but not to human, E. coli or pBR322 DNA, in Southern hybridizations.

**TABLE I**

| | |
|---|---|
| Oligo 85-233: | 5'-CAAAAACTATTCTTAAACCTACCAAGCCTC-3' |
| Oligo 85-241: | 5'-TATTACATTTTAGAATCGCAAAACCAGCC-3' |
| Oligo 86-30; | 5'-TAGGTTTCCCTGAAACATACATATGGTGT-3' |
| Oligo 86-32: | 5'-TGGTCTGCTAGTTCAGGGTCTACTTGTGTGC-3' |
| Oligo 86-34: | 5'-CACCTAGGGCTAACTATGTGTCCTAATAAGG-3' |
| Oligo 86-36: | 5'-TTTCGTAACACTAGGCAAAGGTGGCTTTATC-3' |
| Oligo 86-38: | 5'-TGGTCTTCTGGGGCTTGTTCCATCTATCCTC-3' |
| Oligo 86-40: | 5'-AGGGAAAATGTCTAACAGCTTCATTCTTAAC-3' |
| Oligo 86-42: | 5'-AAATGGATAAACAGCAGTTGTTGCAGAATTC-3' |
| Oligo 86-44: | 5'-TCGAGTAACGCCTATTCTGCTATGTCGACAC-3' |
| Oligo 86-270: | 5'-CTGTGTAATGACTGAGGTGTTACAACTTGT-3' |
| Oligo 86-272: | 5'-TCTAATTACTACCTCTTCTTCTGCTAGACT-3' |
| Oligo 87-84: | 5'-AATATGTTGTTATTACCAATCTAGCAT-3' |

Each of the oligonucleotides can be a DNA or a RNA, although DNAs have been employed in testing. Of course, for the oligonucleotides which are RNAs, the T's in the above-specified sequences represent uridines.

In cases where the analyte to be detected is HIV-1 proviral DNA from infected lymphocytes or DNA reverse transcribed from HIV-1 RNA, oligos with sequences complementary to those of the oligos listed in Table I will also be suitable for probes.

DETAILED DESCRIPTION OF THE INVENTION

In one of its aspects, the present invention is a solid support with a single-stranded oligonucleotide probe of 15 to 100 bases bound thereto and selected from

(A) porous silicate glass derivatized at silicons with

(1) groups of formula

$-(CH_2)_n(NH)(CO)(CH_2)_cR_1$ and
$-(CH_2)_n(NH)(PO_2)O$-(Oligo),

with substantially no silicon derivatized with a group terminated with an amino group, wherein c is 0 to 5, n is 2 to 8, $R_1$ is methyl or benzyl, -O-(Oligo) is the oligonucleotide probe, and the oxygen atom bonded to (Oligo) is the 5'-oxygen of the 5'-nucleoside or the 3'-oxygen of the 3'-nucleoside of the probe; or

(2) groups of formula

$-L_1-CO_2H$ and
(i) $-L_1-(CO)(NH)(CH_2)_pNH(PO_2)O$-(Oligo), or
(ii) $-L_1-(CO)(NH)(CH_2)_pSS(CH_2)_rNH(PO_2)O$-(Oligo),

wherein $-L_1-$ is $-(CH_2)_n(NH)(CO)(CH_2)_m-$,
wherein the group $-(CH_2)_n$ is bonded to a silicon atom; and wherein m, n, p and r are the same or different and are each 2 to 8; or

(3) groups of formula

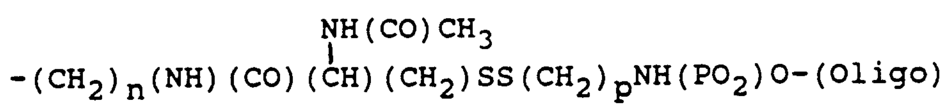

and

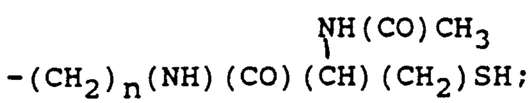

or

(B) a cross-linked dextran macroporous material derivatized at hydroxyl oxygens, which are on carbons of the sugar moieties which have at least one neighboring carbon with an underivatized hydroxyl, with

(1) groups of formula

$-C(=NH)NH(CH_2)_q(NH)(CO)(CH_2)_cR_1$ and
$-C(=NH)NH(CH_2)_q(NH)(CO)(CH_2)_pNH(PO_2)O$-(Oligo)

with substantially no hydroxyl oxygen derivatized with a group terminated with an amino group, or

(2) a group of formula

$-C(=NH)NH(CH_2)_p(NH)(PO_2)O$-(Oligo), or

(3) groups of formula

$-C(=NH)NH(CH_2)_qCO_2H$ and

(i) $L_2$-NH($PO_2$)O-(Oligo),or

(ii) $L_2$-SS($CH_2$)$_r$NH($PO_2$)O-(Oligo)

wherein c,q, p and r are the same or different and q is 2 to 10 and wherein $L_2$ is

-C(=NH)NH($CH_2$)q(CO)(NH)($CH_2$)$_p$-,

wherein the -C(=NH) group is bonded to a hydroxyl oxygen of the dextran; or
(4) groups of formula

-C(=NH)NH($CH_2$)$_n$SH and
-C(=NH)NH($CH_2$)$_n$SS($CH_2$)$_p$NH($PO_2$)O-(Oligo);

and
(C) polystyrene, which may optionally be cross-linked with a cross-linking agent such as divinylbenzene, and which is derivatized at phenyl groups with
(1) groups of formula

-($CH_2$)$_s$$CO_2$H and
(i) $L_3$-NH($PO_2$)O-(Oligo), or

(ii) $L_3$-SS($CH_2$)$_n$NH($PO_2$)O-(Oligo),

wherein $L_3$ is -($CH_2$)$_s$(CO)(NH)($CH_2$)$_p$-,
wherein the ($CH_2$)$_s$ group is bonded to a phenyl group of the polystyrene and wherein s, p and n are the same or different and s is 2 to 10; or
(2) groups of formula

-($CH_2$)$_s$(CO)(NH)($CH_2$)$_p$(NH)(CO)($CH_2$)$_c$$R_1$

and

-($CH_2$)$_s$(CO)(NH)($CH_2$)$_p$(NH)($PO_2$)O-(Oligo),

with substantially no phenyl group derivatized with a group terminated with an amino group; or
(3) groups of formula

-($CH_2$)$_s$(CO)(NH)($CH_2$)$_p$SH, and
-($CH_2$)$_s$(CO)(NH)($CH_2$)$_p$SS($CH_2$)$_n$NH($PO_2$)O-(Oligo).

In another of its aspects, the invention is a method of detecting a single-stranded nucleic acid analyte in a sample, employing:
(a) a solid support, which comprises a first probe ("capture probe"), which is a single-stranded oligonucleotide of between 15 and 100 bases in a sequence complementary to the sequence of a first segment of said analyte, a substantial fraction of said first probe which is part of said solid support being solely end-attached to said support (i.e., bound to said support covalently and only through a linker bonded to either the 5'-oxygen of the 5'-terminal nucleoside or the 3'-oxygen of the 3'-terminal nucleoside), and
(b) a second probe, which is a single-stranded oligonucleotide with a sequence complementary to the sequence of a second segment of said analyte and is labeled for detection, provided that the sequence of the second probe is non-complementary to that of the first probe and the second segment does not overlap the first segment, which method comprises:
(1) incubating the solid support under prehybridizing conditions with a prehybridization solution which comprises (i) a non-homologous nucleic acid, or (ii) dextran sulfate at a concentration between about 5 %(w/v) and 20 %(w/v), or both (i) and (ii);
(2) incubating under hybridizing conditions the solid support, prehybridized in accordance with step (1), with an hybridization solution which comprises between about 5% (w/v) and 20% (w/v) dextran

sulfate; nucleic acid from the sample being assayed, including the analyte if present in said sample; and the second probe, in molar excess relative to the quantity of analyte in the hybridization solution;

(3) after step (2), washing the solid support to remove therefrom second probe that is not stably hybridized to analyte; and

(4) after step (3), determining whether signal due to the detectable label on the second probe hybridized to the analyte is associated with said solid support.

It is to be understood that, prior to step(2) in the method of the invention described in the preceding paragraph, second probe optionally may be incubated in a solution under hybridizing conditions with nucleic acid from the sample being assayed, whereby, if target analyte is present in said sample, at least a fraction of the target analyte will already be hybridized to second probe in the hybridization solution with which solid support is combined in accordance with said step(2) at the time said step(2) is started. That is, solution hybridization of labeled second probe with analyte may be carried out prior to capture of target with capture probe of the solid support.

Another method according to the invention for detecting a single-stranded nucleic acid analyte in a sample, employing:

(a) a solid support, which comprises a first probe ("capture probe"), which is a single-stranded oligonucleotide of between 15 and 100 bases in a sequence complementary to the sequence of a first segment of said analyte, a substantial fraction of said first probe which is part of said solid support being solely end-attached to said support (i.e., bound to said support covalently and only through a linker bonded to either the 5'-oxygen of the 5'-terminal nucleoside or the 3'-oxygen of the 3'-terminal nucleoside), and

(b) a second probe, which is a single-stranded oligonucleotide with a sequence complementary to the sequence of a second segment of said analyte and is labeled for detection, provided that the sequence of the second probe is non-complementary to that of the first probe and the second segment does not overlap the first segment, comprises:

(1) incubating the solid support under prehybridizing conditions with a prehybridization solution which comprises (i) a non-homologous nucleic acid, or (ii) dextran sulfate at a concentration between about 5 %(w/v) and 20 %(w/v), or both (i) and (ii);

(2) incubating under hybridizing conditions the solid support, prehybridized in accordance with step (1), with a first hybridization solution which comprises between about 5% (w/v) and 20% (w/v) dextran sulfate; nucleic acid from the sample being assayed, including the analyte if present in said sample, said first hybridization solution being substantially free of second probe;

(3) after step (2), incubating under hybridizing conditions the solid support with a second hybridization solution which comprises second probe in molar excess relative to the quantity of analyte in said second solution, including analyte bound through hybridization to said first probe to said solid support;

(4) after step (3), washing the solid support to remove therefrom second probe that is not stably hybridized to analyte; and (5) after step (4), determining whether signal due to the detectable label on the second probe hybridized to the analyte is associated with said solid support.

The invention further entails test kits for carrying out the methods according to the invention for detecting a single-stranded nucleic acid analyte in a sample. Said kits comprise a solid support according to the invention, a labeled probe for the analyte to be captured on the solid support, and reagents (if any) required to generate a signal from labeled probe (e.g., enzyme-linked avidin if the label is biotin; flourescence-enhancement solution if the label is chelated europium ion (see Musso et al., PCT International Publication No. WO87/02708)).

In still another aspect, the invention entails a DNA or RNA with the sequence of an oligonucleotide listed in Table I above, or a complement thereof (i.e., an oligonucleotide with sequence complementary to that of such an oligonucleotide), said DNA or RNA optionally labeled for detection. Any of the numerous methods known in the art for labeling an oligonucleotide to make it detectable can be employed with the DNAs and RNAs of the invention. For example, a DNA or RNA can be labeled with $^{32}P$ in the phosphate group at the 5'-carbon of the 5'-nucleoside employing T4 polynucleotide kinase; with biotin linked through a diaminoalkyl linker moiety to a phospohoramidate group at the 5'-terminus of the DNA or RNA (as described by Chu and Orgel, DNA 4, 327-331(1985) or through a linker to a modified nucleoside base (as described, for example, by Dreyer and Dervan, Proc. Natl. Acad. Sci. (USA) 82, 968-972(1985); or with chelated europium ion (as described by Musso et al., PCT International Publication No. WO87/02708).

An oligonucleotide to be employed as a capture probe can be attached to solid-support material by (1) various non-covalent interactions (such as those whereby nucleic acids bind to materials such as nitrocellulose or nylon filters), (2) covalent bonds between reactive groups on the material or on groups covalently derivatized to the material and reactive groups on the pyrimidine or purine base moieties of the

oligonucleotide and (3) covalent bonds between reactive groups on the material or on groups covalently derivatized to the material and a reactive group on a group derivatized to one terminal nucleoside or the other of the oligonucleotide (e.g., through the 5'-oxygen of the 5'-terminal nucleoside or the 3'-oxygen of the 3'-terminal nucleoside). A capture probe is said to be "end-attached" in a solid support when the only means of covalent attachment to the support is of type (3) just described. It is advantageous, particularly for the use of solid supports with capture probe in nucleic acid probe assays, to have a high fraction of the capture probe end-attached, and to minimize non-covalent interactions between the end-attached probe and the support material, because such end-attached capture probe is maximally available to capture target. A "substantial fraction" of capture probe in a solid support is end-attached when more than about 10 % of the capture probe in the support is end-attached. Preferably at least about 30 % is end-attached.

Measurement of extent of end-attachment for solid supports in which capture probe is joined tosolid support materials employed in the present invention (e.g., silicate glass beads, cross-linked dextran, polystyrene) by a linker which entails a disulfide bond or a phosphoramidate group at the terminus of the nucleic acid is possible because the disulfide bond is easily reduced, releasing under mild conditions probe which is end attached but not probe which is covalently attached through one or more bases, and because the phosphoramidate bond is acid labile, also releasing under suitable acidic conditions probe which is end-attached but not probe which is attached covalently through one or more bases. Non-covalent attachment can be estimated by simply incubating capture probe with support material under conditions which do not result in covalent attachment through the linker or otherwise and then measuring the amount of probe absorbed by the material. In these measurements of covalent and non-covalent attachment, $^{32}P$-labeled capture probe is conveniently employed, as the amount of probe associated with and removed from support can then be readily measured.

Particularly from measurements, as described above, of how capture probes are attached to various materials with various linker moieties, the superior properties of solid supports according to the invention encompassed by group (B)(3) described above (cross-linked dextran macroporous material derivatized at certain hydroxyl oxygens with groups of formula

$-C(=NH)NH(CH_2)_qCO_2H$ and

(i) $L_2$-$NH(PO_2)O$-(Oligo) or

(ii $L_2$-$SS(CH_2)_rNH(PO_2)O$-(Oligo))

have been discovered. Most preferred of the dextran materials among these supports is Sephacryl® S-500. The linkers without the disulfide are more preferred.

It should be recognized that macroporous cross-linked dextran materials other than the preferred Sephacryl® S-500 can be employed in the solid supports according to the invention. For example, Sephacryl® S-200 and Sephacryl® S-1000 sold by Pharmacia, Inc. can be employed, as can other macroporous cross-linked dextran materials, particularly such materials made, like Sephacryls®, by cross-linking allyldextran with N,N'-methylenebisacrylamide, sold by Pharmacia as well as other companies.

Although the present invention is illustrated herein with detection of HIV-1, it will be apparent to the skilled that the invention is applicable to detecting any nucleic acid analyte, particularly one which occurs in a sample to be assayed in single-stranded form (i.e., with a large excess of one strand over the strand of complementary sequence), and is not limited to HIV-1.

Further, the solid supports according to the invention, although described herein primarily in terms of their utility in nucleic acid probe assays, can also be employed in affinity chromatography, as "oligo dT columns" have long been employed, to isolate nucleic acids of interest. Whereas oligo-dT columns are limited to isolating poly-A-tailed RNAs, the supports of the present invention can be used to isolate a specific nucleic acid of interest, whether DNA or RNA and whether polyadenylated or not. The supports of the invention in which capture probe is linked to support material by a linker which has a disulfide bond are, in some cases, advantageously employed for obtaining a nucleic acid of interest by affinity chromatography because of the conditions required to separate capture probe (annealed to nucleic acid of interest) from solid support in these supports with disulfide linkers are mild and easy to carry out.

Reference herein to "active esters" is, as understood in the art, to esters formed with carboxylate groups by compunds such as N-hydroxysuccinimide, S-2-pyridine and the like.

A sample to which the assay methods of the invention are applied will usually be obtained by treating a raw specimen, such as blood or other body fluid, a tissue sample from plant or animal, a food sample, a culture of microorganisms, or the like, by methods known in the art to isolate from it nucleic acid, or at least the fraction of nucleic acid thought to include nucleic acid analyte (i.e., "target"). The isolation process will

separate nucleic acid from other substances, such as proteins, that might interfere with the assay, and, in the case of certain enzymes, degrade nucleic acid analyte and oligonucleotide probes.

In certain cases, in which analyte, if present at all, will likely be present at concentrations below the detection limit of the assay, it may be necessary to amplify the amount of analyte, following, for example, procedures adapted from Saiki et al., Science 231, 1434-1436 (1985).

Prehybridizing and hybridizing conditions, and methods of washing to remove unstably hybridized or non-hybridized probe, are well known to the skilled in the nucleic acid probe assay art.

Second probe can easily be provided in molar excess relative to the quantity of analyte in a hybridization solution in the vast majority of cases in which, from experience, the maximum amount of analyte that might be present is known. In case this maximum happens to not be known, several assays, in which second probe concentration varies over a wide range, can be run with different portions of a sample to insure that an assay with a molar excess of second probe is carried out.

Determining whether signal from the label on second probe hybridized to analyte is associated with solid support entails carrying out measurements on the support that are appropriate to the label, to detect any such signal from the support. Preferably, signal from a support to which analyte, from a test sample, might be bound, will be compared with signal from a control support, which is a support treated in substantially the same way as that used with test sample but to which analyte is known not to be bound. A signal from test sample support of greater magnitude than that from control support, as long as it is known that the assay was operative with both supports, confirms that signal from test sample support is due to analyte in the test sample. Of course, as understood in the art, a positive control sample, which is known to contain nucleic acid being assayed for, can also be employed in parallel with test sample and negative control sample to establish that the assay system is operative.

The invention will now be illustrated in some detail in the following examples.

## EXAMPLE I

### PREPARATION AND PURIFICATION OF PROBES

In sandwich assays employing short oligonucleotides, including the component thereof associated with capture of analyte from solution, it is important for sensitivity and specificity, and therefore reliability, that the probes be highly purified and of uniform sequence. While numerous methods of synthesis and purification are available to the skilled to obtain oligonucleotides of sufficient quality, we have found the following to be acceptable: Oligonucleotides are synthesized using an Applied Biosystem 380A automated synthesizer utilizing cyanoethyl phosphoramidite chemistry. Syntheses are carried out on a 1 µmole scale using a short coupling cycle of 9 minutes. Characteristic yields for each coupling are 98%, as measured by trityl detection using a colorimetric assay. Such a procedure synthesizes a 30-nucleotide DNA probe in three hours, followed by an additional 18 hours for removal of base-protecting groups and purification of the tritylated oligonucleotide. The quantities of oligonucleotide obtained at the end of the purification protocol range between 400-600 µg of oligonucleotide per synthesis.

Because pure, full-length probe are a critical requirement for the unambiguous detection of specific target nucleic acid analyte, we have paid particular attention to developing a protocol of oligonucleotide purification. The purification of tritylated oligonucleotides is carried out using C-18 reverse-phase, semipreparative chromatography (10 x 250 mm column). Attempts at isolating oligonucleotides which were detritylated prior to C-18 chromatography proved to be a less efficient means of purification due to the less hydrophobic nature of the oligonucleotides. A gradient of 15-35% acetonitrile in 0.1 M triethylammonium acetate, pH 6.6, over a period of 40 minutes is used to elute the tritylated full-length oligonucleotide. This oligonucleotide is subsequently detritylated using 80% acetic acid in water for one hour. Detritylation is followed by G-50 Sephadex® chromatography. The isolated oligonucleotide is further characterized by HPLC on an RPC-5 column (4.6 x 250 mm column; solvent A, 2 mM Tris, pH 12, solvent B, 2 mM Tris, 200 mM perchlorate, pH 12; gradient 10% B to 50% B over 40 minutes). This last step can also provide an additional step of purification for small quantities of oligonucleotides.

For oligonucleotide probes of longer length (>50 nucleotides), isolation of full-length oligonucleotides is accomplished by preparative polyacrylamide gel electrophoresis using a 12-20% gel after protocols described by Maniatis et al., supra.

The foregoing procedures (for oligonucleotides shorter than 50 nucleotides) were used to prepare the oligonucleotides listed in Table I as DNAs.

EXAMPLE II

PHOSPHORYLATION OF PROBES

Oligonucleotide probes are phosphorylated at the 3'-carbon of the 3'-nucleoside ("3'-terminus") or the 5'-carbon of the 5'-nucleoside ("5'-terminus") using any standard technique.

For phosphorylation at the 3'-terminus, see Panet and Khorana, J. Biol. Chem. 249, 5213-5221.

Oligonucleotides have been routinely phosphorylated at their 5'-termini using T4 polynucleotide kinase following a standard protocol (see Maniatis et al., supra).

For preparing, $^{32}$-P labeled probe, substrates including $^{32}$P-containing phosphate at appropriate positions are employed in the phosphorylation.

EXAMPLE III

LABELING AND DETECTION OF PROBES

Labeling of probe for detection purposes can be accomplished radioactively or non-radioactively.

If radioactive labeling is employed, $^{32}$P labeling as described in Example II is preferred, although labeling with other isotopes, e.g. $^{125}$I or $^{14}$C, following methods known in the art may also be employed.

Scintillation counting is employed to detect radioactively labeled probe complexed with analyte complexed, in turn, to capture probe bound to solid support.

Numerous non-radioactive labeling and associated detection protocols are known. Any of these labeling techniques which involve minimal interference with base-pairing between labeled oligonucleotide probe and the segment of analyte which has sequence complementary to that of the probe can be employed in the present invention. For example, labeling with biotin through a diamine linker joined to biotin and, through a phosphoramidate bond, to a phosphate at the 5'-terminus of the oligonucleotide, following Chu and Orgel, DNA, vol. 4, 327-331 (1985), can be employed, as can similar labeling with EDTA chelated, fluorescent Eu$^{+3}$ in place of biotin, as described in Musso, et al., PCT International Publication No. WO87/02708. Labeling with biotin linked to a nucleoside base, as described by Dreyer and Dervan, supra, can also be employed.

EXAMPLE IV

DERIVATIZATION OF POROUS SILICATE GLASS

(A) "Longchain alkylamine" derivatized controlled pore glass was purchased from Pierce Chemical Co., Rockford, Illinois, U.S.A., Product No. 24875. This porous silicate glass has silicons derivatized with a group of formula $-(CH_2)_6NH_2$.

(B) Carboxyl-derivatized controlled pore glass was purchased from Pierce Chemical Co., Rockford, Illinois, U.S.A., Product No. 23735. This porous silicate glass has silicons derivatized with a group of formula $-(CH_2)_3NH(CO)(CH_2)_2CO_2H$.

(C) Thiol-derivatized controlled pore glass was also purchased from Pierce Chemical Co., Rockford, Illinois, U.S.A., Product No. 23748. This porous silicate glass has silicons derivatized with a group of formula

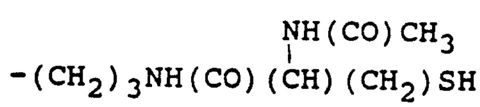

(D)Succinimido-ester derivatized porous silicate glass is prepared with the carboxyl-derivatized glass described above. The carboxyl-derivatized glass is dried by washing three times with acetone, followed by three times with dry acetone (dried by sitting over four Angstrom molecular sieves). The beads are then filtered using a sintered glass funnel and dried for 10 hours in vacuo. To 500 mg of the dry beads in 15 ml of dry N,N-dimethyl-formamide ("DMF") are added 500 mg dicyclohexyl carbodimide and 300 mg N-hydroxysuccinimide. The mixture is agitated for 16 hours and washed with dry DMF (3x, 20 ml each) and methylene chloride (2x, 20 ml each) to remove excess reagent and dicyclohexyl-urea. The resulting support is then dried for three house in vacuo.

EXAMPLE V

DERIVATIZATION OF MACROPOROUS CROSS-LINKED DEXTRAN SUPPORT

(A) Sephacryl® S-500 was purchased from Pharmacia Inc., Piscataway, New Jersey, USA under Code No. 17-0475-01.

(B) The Sephacryl® S-500 was cyanogen bromide-activated following Bunemann et al., supra. 100 g of wet material as supplied by the manufacturer was washed using a large excess of distilled water and a sintered glass filter. The wet cake was transferred to a 1000 ml beaker equipped with an overhead paddle stirrer and a pH-electrode. 500 ml of ice-cold water was added, and the suspension was stirred vigorously while being kept below 10 °C by occasional addition of pieces of ice. 40 g of solid cyanogen bromide was added, and the pH was monitored repeatedly and maintained between 10.5 and 11.5 by addition of 5M NaOH for about 20 minutes. Then the bead material was washed quickly with five volumes of ice-cold water, followed by one volume of ice-cold 10mM potassium phosphate, pH 8.0.

As the skilled will understand, CNBr activation requires hydroxyl groups on neighboring carbon atoms.

(C) Carboxyl-derivatized material was prepared as follows: To a suspension of 20 g of freshly prepared CNBr-activated Sephacryl® S-500 in 200 ml of 10 mM potassium phosphate, pH 8.0, were added 16 g of 6-aminocaproic acid, in four portions at intervals of 30 minutes, and the resulting mixture was stirred with a paddle stirrer for 20 hours. (Alternatively, hexylenediamine can be used in place of 6-aminocaproic acid to yield the respective amine-derivatized support.)

The resulting derivatized Sephacryl® was filtered through a sintered glass funnel and washed exhaustively with 10 mM potassium phosphate buffer, pH 8.0; 1 M potassium phosphate buffer, pH 8.0; 1M KCl, 0.1M NaOH, and finally water. The wet cake was then suspended in 0.1M MES, pH 6.0, and stored at 4C.

(D) Succinimide-derivatized macroporous crosslinked-dextran material was prepared from the carboxyl-derivatized material as follows: Carboxy-derivatized Sephacryl® S-500, in suspension in 0.1M MES buffer, pH 6, was transferred to a sintered glass funnel and was washed successively with water, 70:30, 50:50 and then 30:70 $H_2O$/acetone, followed by three washes with acetone, followed in turn by three washes with acetone that had been dried over 0.4nm (four Angstrom) molecular sieves. After filtering, the Sephacryl® was dried for ten hours under high vacuum (0.01 torr). Then 500 mg of the dried, carboxyl-derivatized material was treated, as described in Example IV (D) for carboxyl-derivatized porous glass, to yield the succinimide-ester derivatized Sephacryl®.

(E) Thiol-derivatized Sephacryl® S-500 is prepared as follows: To a suspension of 15 g of freshly prepared CNBr-activated Sephacryl® S-500 in 10 mM potassium phosphate, pH 8.0, is added 5 g of cystamine dihydrochloride and the mixture is stirred with a paddle stirrer for 20 hr. The resulting derivatized Sephacryl® is filtered and washed as in V(C). Prior to use, the disulfide bond of the cystamine linker of the derivatized material is cleaved by suspension of the material in 100 mM dithiothreitol (DTT), 50 mM HEPES, pH7.7 for 1 hr. at 25 °C followed by extensive washing of the material with degassed 0.2 M HEPES, pH 7.7, 0.1 M KCl and finally 0.2 M HEPES, pH 7.7 again.

EXAMPLE VI

DERIVATIZATION OF DIVINYLBENZENE-CROSSLINKED POLYSTYRENE SUPPORT

(A) 1 % cross-linked divinylbenzene-polystyrene chloromethyl resin (1.1 mM cl/g, 200 - 400 mesh) was purchased from U. S. Biochemical Corp. (Cleveland, Ohio, USA). 12% divinylbenzene-crosslinked polystyrene beads, mesh size 200-400, were purchased from Polysciences, Warrington, Pennsylvania.

(B) The 12 % crosslinked divinylbenzene-polystyrene beads were carboxy-derivatized as follows:

10g of the beads were added to 150 ml of 1,2-dichloroethane, and the mixture was brought to a reflux. A filtered mixture of 20 mmoles of aluminum chloride and 10 mmoles of glutaric acid monomethyl ester chloride in 10 ml of nitrobenzene was added over a five-minute period, and the reaction mixture was refluxed for an additional four hours. The resin was filtered through a sintered glass funnel, washed with dioxane:4M HCl (3:1), dioxane:water (3:1), dioxane, ethanol, acetone and finally methanol, and then dried in a dessicator under vacuum. To 2 g of KOH in a three-necked flask was added 100 ml of ethylene glycol, and the mixture was heated to 100 °C. When the KOH was fully dissolved, 5 g of the dried, derivatized resin were added, along with 3 ml of hydrazine hydrate. The mixture was brought to a reflux and refluxed for three hours, after which a portion of the liquid was distilled off until the reflux

temperature reached 198 °C. The flask was then stoppered and reflux continued for 12 hours. The resin was then washed as described above (after the derivatization with glutaric acid monomethyl ester chloride).

(C) The 12 % divinylbenzene-crosslinked polystyrene beads were amino-derivatized as follows: To 5 g of carboxyl-derivatized beads were added 1 g of dicyclohexylcarbodiimide and 1.25 g of N-hydroxysuccinimide in 50 ml of dimethylformamide (DMF). After stirring for 30 min., 7 g of 1,8 diaminooctane was added and the mixture was stirred for three more hours. The resin was then filtered, and then washed successively with methanol, water, methanol, $CH_2Cl_2$, and finally acetone.

(D) The 1 % divinylbenzene-crosslinked, chloromethyl-derivatized polystyrene resin was amino-derivatized as follows: To 10 g of the 1 % cross-linked, chloromethyl-derivatized resin (as supplied by U. S. Biochemicals, Inc.) in 80 ml of DMF were added 2.6 g of N-t-butyloxycarbonyl-6-amino caproic acid and 1.28 g of potassium fluoride, and the mixture was stirred at 80 °C for 16 hours. The resin was then filtered, washed successively with $CH_2Cl_2$, methanol and acetone, and then subjected to high vacuum (0.01 torr) for three hours. Deprotection of the amino group was carried out with 30 % trifluoroacetic acid in $CH_2Cl_2$ to yield the amino-derivatized support. Picric acid titration of the amino groups gave a substitution level of 0.17 mmol/g resin.

EXAMPLE VII

5'-AMINO ALKYL, 5'-CARBOXYALKYL, AND 5'-CYSTAMINYL PHOSPHORAMIDATE DERIVATIVES OF OLIGONUCLEOTIDES

(A) 5'-Aminoalkyl Phosphoramidate Derivatives

The aminoalkyl derivatives were typically formed in 1-10 nmole scale at concentrations of 1-20 micromolar. The protocol followed was that of Chu, et al., Nucl. Acids Res. 11, 6513-6529 (1983), and the modified oligonucleotide was isolated by ethanol precipitation, following Chollet and Kawashima, Nucl. Acids Res. 13, 1529-1541 (1985).

Thus, to prepare the amino-hexyl adduct of an oligonucleotide, 4 nmoles of the oligonucleotide (phosphorylated at the 5'-terminus) in a siliconized eppendorf tube were taken up in 0.3 ml of 0.25 M 1,6-diaminohexane, 0.1M N-methylimidazole, and 0.1 M 1-ethyl-3-(3-dimethylaminopropyl) carbodiimide ("CDI"), pH 6.0, and allowed to react for 20 hours. The resulting DNA was ethanol-precipitated three times to remove excess diamine and was then taken up in 0.1 M MOPS, pH 7.5. Prior treatment of reaction tubes with a silanizing solution was found to be essential anytime oligonucleotide is involved in a reaction to prevent non-specific binding of the oligonucleotide to the sides of the tube during the reaction.

Following the same procedure, but with 1,2-diaminoethane or 1,8-diaminooctane in place of 1,6-diaminoohexane, yields the 2-aminoethyl or 8-aminooctyl adduct, respectively, in place of the 6-aminohexyl.

(B) 5'-Carboxyalkyl Phosphoramidate Derivatives

To prepare the title derivatives with glycine or aminocaproic acid, the following procedure was employed: The 5'-phosphorylated oligonucleotide (4 nmol) in a silanized eppendorf tube was taken up in 0.3 ml of 0.1 M imidazole, 0.1 M CDI, pH 6.0, at 25 °C, and the phosphorimidazolide was allowed to form over a period of 90 minutes. The oligonucleotide was then ethanol-precipitated and then redissolved in a 0.3 ml solution of 0.1 M glycine or 0.1 M aminocaproic acid, pH 8, and heated for 3 hours at 50 °C. The resulting adduct was then isolated by ethanol-precipitation and finally taken up in 0.05 M MOPS, pH 7.5.

(C) 5'-Cystaminyl Phosphoramidate Derivatives

To 1 nmole of a 5'-phosphorylated oligonucleotide in a silanized eppendorf tube was added 0.3 ml of 0.1 M imidazoile and 0.15 M CDI, pH 6.00, and the resulting solution was incubated at 23 °C for 1.5 hours. The 5'-imidazolide derivative was isolated by ethanol-precipitation and then taken up in 0.3 ml of 0.25 M cystamine, pH 7.7, whereupon the reaction was allowed to proceed for 3 hours at 50 °C. The cystaminyl phosphoramidate derivative was then ethanol precipitated three times to remove excess cystamine. The DNA pellet was then taken up in 0.05 M MOPS, pH 7.5.

(D) 3'-Derivatives

Following the same procedures as in Examples VII(A), (B) and (C) with oligonucleotides that are phosphorylated at the 3'-terminus, in place of those phosphorylated at the 5'-terminus, yields the 3'-derivatized phosphoramidate adducts in place of the 5'-derivatized.

EXAMPLE VIII

DERIVATIZING AMINO-DERIVATIZED POROUS SILICATE GLASS SUPPORTS WITH OLIGONUCLEOTIDES

The standard reaction that has been used for the title derivatization is as follows:

10 mg of long chain alkyl amine derivatized controlled pore glass (Pierce Chemical Co., product No. 24875), with 20-30 micromoles amino group per gram of glass, are combined in a silanized eppendorf tube with 0.11 ml of 0.1 M N-methylimidazole, 0.1 M CDI, and from 5 x $10^{-7}$ to 20 x $10^{-7}$ M of 5'-phosphorylated oligonucleotide. The reaction is continued for 24 hours, with agitation, and then the beads are given one minute washes with NaOH, pH 12, to remove non-specifically bound DNA. Finally, the derivatized beads are stored in TE buffer (10 mM Tris, 1 mM EDTA, pH 8.0).

Prior to use, the unreacted amino groups of the beads are blocked by mixing 10 mg of beads with 100 µl of 50 mM N-hydroxysuccinimide ester of acetic or propionic acid, 0.2M HEPES, pH 7.7 and reacting for 15 minutes followed by three washes with 0.2M HEPES, pH 7.7 and, finally, storage in TE buffer. This treatment suffices to block substantially all free amino groups in the beads.

Coupling yields of oligonucleotides to the amine-derivatized porous silicate glass beads were determined by reacting 25 pmoles of $^{32}P$-labelled 5' phosphorylated oligonucleotide with 50 mg of the beads in 0.55 ml of 0.1 M methylimidazole, o.1 M CDI, pH 6.00, for 24 hours with agitation, followed by 1 minute washes with NaOH, pH 12, to remove unbound oligonucleotide. Non-specific (i.e., non-covalent) binding was determined by carrying out the reaction in the absence of CDI. Cerenkov counts from the beads were then determined in a Beckman LS 7800 liquid scintillation system (Beckman Instruments, Inc.). The "attachment yield" is the fraction of the 25 pmoles attached to the beads. Attachment yields were found to be a function of the length of the oligonucleotide. Typically, 75-80% yields for a 17-mer were obtained for the amine-derivatized porous glass beads; 40-45% yields were observed for a 25-mer. The extent of non-specific binding varied between 4-6%. Essentially complete detachment of oligonucleotide from the support resulted upon acid hydrolysis of the phosphoramidate bond, indicating that all the oligonucleotide that is not attached non-specifically by the procedure described in this paragraph is end-attached.

EXAMPLE IX

DERIVATIZING CYANOGEN BROMIDE-ACTIVATED MACROPOROUS CROSS-LINKED DEXTRAN SUPPORTS WITH 5'-AMINOALKYLPHOSPHORAMIDATE-DERIVATIZED OLIGONUCLEOTIDE

50 mg of wet, freshly prepared CNBr-activated Sephacryl® S-500 beads are combined with 0.5 ml of 0.1 M $NaHCO_3$, 0.5 M NaCl, pH 8.3, and 0.025 ml of 5'-6-amino-hexylphosphoramidate-derivatized oligonucleotide (approximately 5 x $10^{-6}$ M) in 0.0.05 M MOPS, pH 7.5. The reaction is allowed to proceed for 20 hours. The tube is then centrifuged and supernatant removed, and the beads are then combined with 0.1 M Tris, pH 8.00 for one hour to inactivate unreacted sites remaining from the cyanogen bromide derivatization. The beads are then washed four times with NaOH, pH 12, and finally stored in TE buffer. The linker joining the Sephacryl® hydroxyl oxygen and 5'-oxygen of 5'-nucleoside of the oligonucleotide then has the formula

$-C(=NH)NH(CH_2)_6NH(PO_2)-$.

The same procedure, followed with 2-aminoethyl phosphoramidate-derivatized, in place of 6-aminohexyl phosphoramidate-derivatized, oligonucleotide yields solid support wherein the linker between dextran oxygen and 5'-oxygen of 5'-nucleoside of oligonucleotide has the formula

$-C(=NH)NH(CH_2)_2NH(PO_2)-$

EXAMPLE X

DERIVATIZATION OF CARBOXYL-DERIVATIZED SOLID SUPPORT MATERIALS WITH 5'-AMINOALKYLPHOSPHORAMIDATE-DERIVATIZED OLIGONUCLEOTIDE

The following protocol has been applied with the above-described carboxyl-derivatized porous silicate glass, divinylbenzene cross-linked polystyrene, and macroporous cross-linked dextran supports:

To 50-75 mg of carboxyl-derivatized support (wet weight, in the case of Sephacryl®) is added 25-30 pmoles of the 5'-aminoalkylphosphoramidate-or 5'-cystaminyl phosphoramidate-derivatized oligonucleotide in 0.75 ml of 0.15 M CDI, 0.1 M MES, pH 6, and the mixture is gently agitated in a rotary mixer for one hour (in the case of Sephacryl®) or 16 hours (in the case of glass and polystyrene). (The greater speed of the reaction with the Sephacryl is a marked advantage of that material.) After the reaction, the support is washed four times with NaOH, pH 12, and finally the oligonucleotide-derivatized material is stored in TE buffer.

Coupling yields were obtained for these carboxy-derivatized supports by using $^{32}P$-labelled oligonucleotide and measuring Cerenkov counts of beads, as indicated in the previous example. Non-specific binding was assessed by measurement of radioactivity in the support material after running the coupling reaction in the absence of CDI. Attachment through amino residues of bases rather than through the terminal amino group of the linker bonded to the oligonucleotide was assessed by reacting phosphorylated, but not otherwise derivatized, oligonucleotide with the support material. The extent of end-attachment was obtained from the acid hydrolysis of the phosphoramidate bond in HCl, pH 2, at 37 °C for 4 hours, followed by washes of the support with NaOH, pH 12, whereby the residual radioactivity on the support provided an estimate of the end-attachment. For supports with the 5'-cystaminyl phosphoramidate-derivatized oligonucleotide, the extent of end-attachment was measured by cleavage of the disulfide bond with DTT. Thus, 50 mg of oligonucleotide-derivatized support was incubated for 1 hour at 23 °C with 0.2 ml of 0.1 M DTT, 0.1 M HEPES, and 1 mM EDTA, pH 7.7. The support was then washed with NaOH, pH 12,to remove dissociated oligonucleotide. Generally, the results for end-attachment with the cystaminyl-derivatized oligonucleotides were in agreement with those from the aminoalkyl-derivatized oligonucleotides.

The mild conditions employed to remove the cystaminyl-derivatized oligonucleotides are noteworthy for application of the solid supports of the invention for isolating nucleic acids.

The most preferred solid support of the invention is carboxyl-derivatized Sephacryl® S-500 derivatized with 5'-(6-aminohexyl phosphoramidate)-derivatized oligonucleotide.

With the procedure of this example, about 1.2 pmole of oligonucleotide is derivatized per milligram (wet weight) of carboxyl-derivatized Sephacryl S-500. About half of the oligonucleotide is solely end-attached (covalently only through formation of an amide bond between the 6-amino of the 6-amino-hexylphosphoramidate linked to the oligonucleotide and the carboxyl linked to the Sephacryl®, only about 0.5% is non-covalently attached to the support, and about half is suspected to be covalently attached through amino groups of bases in the oligonucleotide (e.g., cytosines) (possibly together with formation of amide between the carboxyl of the support and the amino of 6-aminohexyl-phosphoramidate).

Higher levels of end attachment and reduced attachment through nucleoside bases are obtained when the carbonyl groups of the support material are activated as their N-hydroxysuccinamide esters. A typical procedure for immobilization suspends 10 mg of dry N-hydroxysuccinimide activated Sephacryl® support in 0.7 ml of 0.2M HEPES pH 7.7, containing 20 - 25 pmoles of a 5'-aminoalkyl phosphoramidate- or 5'-cystaminyl phosphoramidate-derivatized oligonucleotide, and the mixture is agitated for one hr on a rotary mixer. The support is then washed four times with NaOH, pH 12.0 and then stored in TE. (Upon hydration, 50 mg of wet support is obtained from 10 mg of dry material.) Immobilization under these conditions has given 50-55% attachment efficiency and greater than 80% end attachment of oligonucleotide. Results have tended to be variable with the N-hydroxysuccinimide-activated support materials, however. This appears to be due to the susceptibility of the succinimido-ester moiety to hydrolysis. Thus, rigorous exclusion of moisture during the preparation of the succcinimido-ester activated supports and storage of the vacuum dried form under $N_2$ at 4 °C are helpful in assuring good results with the supports.

EXAMPLE XI

DETECTION OF HIV-1 GENOME

This example illustrates a method for using the oligonucleotide-derivatized solid supports of the invention to detect a nucleic acid analyte, in particular the genome of HIV-1 virus, the causative agent of acquired immune deficiency syndrome in humans.

A solid support ("beads") according to the invention was prepared with carboxyl-derivatized Sephacryl® S-500 and 5'-(6-aminohexyl)phosphoramidate derivatized oligonucleotide 85-241 (capture probe). The linker between the dextran oxygen and 5'-oxygen of the 5'-thymidine of the oligonucleotide has the formula

$-C(=NH)NH(CH_2)_5(CO)(NH)(CH_2)_6NH(PO_2)-$.

Oligonucleotide 85-233 was 5'-phosphorylated with $^{32}P$-containing phosphate employing T4 polynucleotide kinase for use as the second, labeled probe.

It should be noted that the procedure that follows could be carried out as well with pairs of oligonucleotides other than 85-241 and 85-233 which have sequences complementary to that of non-overlapping segments of the HIV-1 genome. For example, any pair of oligonucleotides listed in Table I could be employed. Further, it could be carried out with other solid support materials used to make the beads and with a non-radioactive label for detection on the second probe.

Total nucleic acid from the lymphocyte fraction of 10 ml of blood from a person being tested for HIV-1 infection is isolated employing any standard procedure. A sample of 10 µg of human DNA (about $10^8$ genome equivalents) is assayed in parallel as a negative control with the nucleic acid sample being tested for HIV-1 genome. The following protocol is then followed with the sample and the control:

Hybridization solution (HS; 5x SSPE (0.75 M NaCl, 50 mM $NaH_2PO_4$, pH 7.4, 5 mM EDTA), 10% dextran sulfate, and 0.1% sodium dodecyl sulphate (SDS)) is place at 37° C to warm up prior to use.

20x SSC is diluted to 2x SSC (0.3M NaCl, 30 mM sodium citrate, pH 7. 0) and placed at 37° C for later use.

A stock solution of beads is resuspended in TE buffer, and 250 µl of this suspension is removed to obtain 50 mg (wet weight) of beads. This 250 µl is then placed into an eppendorf tube and the beads centrifuged (5 seconds) to pellet the 50 mg of beads. Each assay should be done with 50 mg of beads.

Prehybridization is then carried out as follows: Remove the TE buffer in which the beads are stored after centrifuging for 5-10 seconds. Add 250 µl of prewarmed HS (at 37° C) to beads and resuspend the beads. Place this prehybridization mixture at 37° C for 15-60 minutes.

Sandwich hybridization is then carried out:

Solution Hybridization Step:

While the beads are prehybridizing, add labeled oligonucleotide (second probe) to an aliquot of sample (or control) nucleic acid. The volume of this hybridization solution should not exceed 50 µl and can be done in TE buffer. The ratio of quantity of probe to quantity of target should be about 5:1 (mole to mole).

Transfer the labeled probe-target hybridization solution (50 µl) to 65° C for 5 minutes.

Move the labeled probe-target solution to 42° C. Then add HS to make a final hybridization solution of 250 µl and allow the hybridization to continue for 30 - 60 minutes at 42 ° C.

Capture Step:

Just before using beads for capture, remove the beads from prehybridization temperature, centrifuge the beads to bottom of tube, and remove prehybridization solution. Then place beads at 37° C and transfer the 250 µl hybridization solution (target and labeled probe) to the beads. Resuspend the beads in the hybridization solution and shake every 5 minutes. Keep at 37° C for 60 minutes.

Washes:

While beads are capturing hybridization complex, set up scintillation vials and pipettes.

After 60 minutes for capture, centrifuge beads (5-10 seconds) and remove hybridization solution.

Add 1 ml of prewarmed (37° C) 2x SSC to the beads, shake tube, and centrifuge for 5-10 seconds. Remove the wash solution and repeat the wash twice more.

After removing the last wash, count the beads for each sample employing the scintillation counter (e.g., a Beckman LS7800).

Elution of Labeled Probe from Beads:

To recover the isotopically labeled probe from the beads, the beads are resuspended in 100-1000 µl of water and heated to 100° C for 5 minutes.

The suspension is transferred to dispo columns (Quik-Sep) and spun to collect the elution solution.

This solution should contain 80% of label associated with the beads. The label can be concentrated by lyophilization.

By subtracting background counts obtained from the solid support used with human DNA negative control, from the counts arising from the solid support with sample nucleic acid, thought to contain analyte,

the signal from the solid support with sample nucleic acid, due to label on the oligonucleotide hybridized to analyte, is determined.

As indicated above, the use of dextran sulfate (at 5%-20% concentration, preferably about 10%) in at least the capture hybridization have been found to markedly enhance the signal to noise ratio in the use of solid supports of the present invention to detect nucleic acid analyte. This has been the case with the beads employed in the present example to detect HIV-1 genome as analyte.

As further indicated in the present example, use of dextran sulfate (at 5%-20% concentration, preferably about 10%) in the prehybridization solution has also been found to be advantageous in increasing the signal to noise ratio in the use of solid supports of the invention to detect nucleic acid analyte.

In a less preferred modification of the foregoing procedure of this example, a non-homologous (also referred to in the present specification as "inert") nucleic acid, such as sonicated salmon sperm DNA, can be included in the prehybridization solution at a concentration of about 1 mg/ml.

## Claims

**1.** A method of detecting a single-stranded nucleic acid analyte in a sample, employing:

(a) a solid support, which comprises a first probe, which is a single-stranded oligonucleotide of between 15 and 100 bases in a sequence complementary to the sequence of a first segment of said analyte, a substantial fraction of said first probe which is part of said solid support being bound to said support covalently and only through a linker bonded to either the 5'-oxygen of the 5'-terminal nucleoside of the 3'-oxygen of the 3'-terminal nucleoside, and

(b) a second probe, which is a single stranded oligonucleotide with a sequence complementary to the sequence of a second segment of said analyte and is labeled for detection, provided that the sequence of the second probe is non-complementary to that of the first probe and the second segment does not overlap the first segment, which method comprises:

(1) incubating the solid support under prehybridizing conditions with a prehybridization solution which comprises (i) a non-homologous nucleic acid, or (ii) dextran sulfate at a concentration between about 5 %(w/v) and 20 %(w/v), or both (i) and (ii);

(2) incubating under hybridizing conditions the solid support, prehybridized in accordance with step (1), with an hybridization solution which comprises between about 5% (w/v) and 20% (w/v) dextran sulfate; nucleic acid from the sample being assayed, including the analyte if present in said sample; and the second probe, in molar excess relative to the quantity of analyte in the hybridization solution;

(3) after step (2), washing the solid support to remove therefrom second probe that is not stably hybridized to analyte; and

(4) after step (3), determining whether signal due to the detectable label on the second probe hybridized to the analyte is associated with said solid support.

**2.** A method according to Claim 1 wherein the solid support with first probe bound thereto is selected from:

(A) porous silicate glass derivatized at silicons with

(1) groups of formula

$-(CH_2)_n(NH)(CO)(CH_2)_cR_1$ and
$-(CH_2)_n(NH)(PO_2)O\text{-(Oligo)}$,

with substantially no silicon derivatized with a group terminated with an amino group, wherein c is 0 to 5, n is 2 to 8, $R_1$ is methyl or benzyl, -O-(Oligo) is the oligonucleotide probe, and the oxygen atom bonded to (Oligo) is the 5'-oxygen of the 5'-nucleoside or the 3'-oxygen of the 3'-nucleoside of the probe; or

(2) groups of formula

$-L_1-CO_2H$ and
(i) $-L_1-(CO)(NH)(CH_2)_pNH(PO_2)O\text{-(Oligo)}$, or

(ii) $-L_1-(CO)(NH)(CH_2)_pSS(CH_2)_rNH(PO_2)O\text{-(Oligo)}$,

wherein $-L_1-$ is $-(CH_2)_n(NH)(CO)(CH_2)_m-$,

wherein the group $-(CH_2)_n$ is bonded to a silicon atom; and wherein m, n, p and r are the same or different and are each 2 to 8; or
(3) groups of formula

$$-(CH_2)_n(NH)(CO)(\overset{NH(CO)CH_3}{\overset{|}{CH}})(CH_2)SS(CH_2)_pNH(PO_2)O\text{-}(Oligo)$$

and

$$-(CH_2)_n(NH)(CO)(\overset{NH(CO)CH_3}{\overset{|}{CH}})(CH_2)SH;$$

or
(B) a cross-linked dextran macroporous material derivatized at hydroxyl oxygens, which are on carbons of the sugar moieties which have at least one neighboring carbon with an underivatized hydroxyl, with
(1) groups of formula

$-C(=NH)NH(CH_2)_q(NH)(CO)(CH_2)_cR_1$ and
$-C(=NH)NH(CH_2)_q(NH)(CO)(CH_2)_pNH(PO_2)O$-(Oligo)

with substantially no hydroxyl oxygen derivatized with a group terminated with an amino group, or
(2) a group of formula

$-C(=NH)NH(CH_2)_p(NH)(PO_2)O$-(Oligo), or

(3) groups of formula

$-C(=NH)NH(CH_2)_qCO_2H$ and

(i) $L_2-NH(PO_2)O$-(Oligo),or

(ii) $L_2-SS(CH_2)_rNH(PO_2)O$-(Oligo)

wherein c,q, p and r are the same or different and q is 2 to 10 and wherein $L_2$ is

$-C(=NH)NH(CH_2)_q(CO)(NH)(CH_2)p-$,

wherein the $-C(=NH)$ group is bonded to a hydroxyl oxygen of the dextran; or
(4) groups of formula

$-C(=NH)NH(CH_2)_nSH$ and
$-C(=NH)NH(CH_2)_nSS(CH_2)_pNH(PO_2)O$-(Oligo);

and
(C) polystyrene, which may optionally be cross-linked with a cross-linking agent such as divinylbenzene, and which is derivatized at phenyl groups with
(1) groups of formula

$-(CH_2)_sCO_2H$ and

(i) $L_3-NH(PO_2)O$-(Oligo), or

(ii) $L_3$-$SS(CH_2)_nNH(PO_2)O$-(Oligo),

wherein $L_3$ is $-(CH_2)_s(CO)(NH)(CH_2)_p-$, wherein the $(CH_2)_s$ group is bonded to a phenyl group of the polystyrene and wherein s, p and n are the same or different and s is 2 to 10; or
(2) groups of formula

$-(CH_2)_s(CO)(NH)(CH_2)p(NH)(CO)(CH_2)_cR_1$

and

$-(CH_2)_s(CO)(NH)(CH_2)_p(NH)(PO_2)O$-(Oligo),

with substantially no phenyl group derivatized with a group terminated with an amino group; or
(3) groups of formula

$-(CH_2)_s(CO)(NH)(CH_2)_pSH$, and
$-(CH_2)_s(CO)(NH)(CH_2)_pSS(CH_2)_nNH(PO_2)O$-(Oligo).

**3.** A method according to Claim 2 wherein the first probe is bound to the solid support through the 5'-oxygen of the 5'-nucleoside and wherein the solid support with first probe bound thereto is selected from:
(A) a controlled pore glass, in the form of beads of diameter between about 100 and about 200 $\mu$m (microns) and pore diameter between about 0.04 $\mu$m and 0.06 $\mu$m (400-600 Angstroms) and derivatized at silicons with groups of formula

$-(CH_2)_a(NH)(CO)(CH_2)_bCH_3$ and
$-(CH_2)_a(NH)(PO_2)O$-(Oligo)

with substantially no silicon derivatized with a group terminated with an amino group, wherein a is 3 or 6 and b is 0, 1, or 2; and
(B) a macroporous material, made by cross-linking allyldextran with N,N'-methylenebis-acrylamide, in the form of beads with a wet diameter between about 30 and about 120$\mu$m (microns) and an exclusion volume for dextrans between about $10^6$ daltons and about $10^8$ daltons, and derivatized, at hydroxyl oxygens, which are on carbons of the sugar moieties which have at least one neighboring carbon with an underivatized hydroxyl, with groups of formula

$-C(=NH)NH(CH_2)_6NH(CO)(CH_2)_bCH_3$ and
$-C(=NH)NH(CH_2)_6NH(CO)(CH_2)_pNH(PO_2)O$-(Oligo),

or

$-C(=NH)NH(CH_2)_6NH(PO_2)O$-(Oligo),

with substantially no hydroxyl oxygen derivatized with a group terminated with an amino group.

**4.** A method according to Claim 2 wherein the first probe is bound to the solid support through the 5'-oxygen of the 5'-nucleoside and wherein the solid support with first probe bound thereto is selected from:
(A) a controlled pore glass, in the form of beads of diameter between about 100 and about 200 $\mu$m (microns) and pore diameter between about 0.04 and 0.06 $\mu$m (400-600 Angstroms) and derivatized at silicons with groups of formula

$-(CH_2)_3NH(CO)(CH_2)_2CO_2H$ and
$-(CH_2)_3NH(CO)(CH_2)_2(CO)(NH)(CH_2)_6NH(PO_2)O$-(Oligo);

and

(B) a macroporous material, made by cross-linking allyldextran with N,N'-methylenebisacrylamide, in the form of beads with a wet diameter between about 30 and about 120µm (microns) and an exclusion volume for dextrans between about $10^6$ daltons and about $10^8$ daltons, and derivatized at hydroxyl oxygens, which are on carbons of the sugar moieties which have at least one neighboring carbon with an underivatized hydroxyl, with groups of formula

$-C(=NH)NH(CH_2)_5CO_2H$ and
$-C(=NH)NH(CH_2)_5(CO)(NH)(CH_2)_6NH(PO_2)O-(Oligo)$

and
(C) 0% to about 16% divinylbenzene-crosslinked polystyrene beads, of between about 60 and 500 mesh size, derivatized at polystyrene phenyls with groups of formula

$-(CH_2)_4CO_2H$ and
$-(CH_2)_4(CO)(NH)(CH_2)_6(NH)(PO_2)O-(Oligo)$.

**5.** A method according to Claim 3 or 4 wherein both the first probe and the second probe have 25-50 bases and the second probe is radioactively labeled.

**6.** A method according to Claim 5 wherein the radioactive label on the second probe is $^{32}P$ of a phosphate group bonded to the 5'-carbon of the 5'-nucleoside.

**7.** A method according to Claim 6 wherein both the prehybridization solution and the hybridization solution comprise between about 8 (w/v) and about 12 %(w/v) dextran sulfate.

**8.** A method according to Claim 7 wherein, prior to step (2) according to Claim (1), nucleic acid from the sample being assayed is incubated under hybridizing conditions in a first hybridization solution, which comprises a molar excess, relative to the concentration of analyte in the hybridization solution, of second probe, until a substantial fraction of any analyte that is present has hybridized to second probe, and then step (2) according to Claim 1 is initiated by combining with said first hybridization solution the solid support prehybridized according to step (1) of Claim 1 and immersed in a second hybridization solution.

**9.** A method according to any of Claims 5 to 8 wherein the analyte is the genome of an HIV-1 virus, or a fragment thereof which includes segments with sequences complementary to the first probe and the second probe.

**10.** A solid support for use in the method of any of Claims 1 to 9 with a single-stranded oligonucleotide probe of 15 to 100 bases bound thereto and selected from
(A) porous silicate glass derivatized at silicons with
(1) groups of formula

$-(CH_2)_n(NH)(CO)(CH_2)_cR_1$ and
$-(CH_2)_n(NH)(PO_2)O-(Oligo)$,

with substantially no silicon derivatized with a group terminated with an amino group, wherein c is 0 to 5, n is 2 to 8, $R_1$ is methyl or benzyl, -O-(Oligo) is the oligonucleotide probe, and the oxygen atom bonded to (Oligo) is the 5'-oxygen of the 5'-nucleoside or the 3'-oxygen of the 3'-nucleoside of the probe; or
(2) groups of formula

$-L_1-CO_2H$ and

(i) $-L_1-(CO)(NH)(CH_2)_pNH(PO_2)O-(Oligo)$, or

(ii)$-L_1-(CO)(NH)(CH_2)_pSS(CH_2)_rNH(PO_2)O-(Oligo)$,

wherein $-L_1-$ is $-(CH_2)_n(NH)(CO)(CH_2)_m-$,

wherein the group $-(CH_2)_n$ is bonded to a silicon atom; and wherein m, n, p and r are the same or different and are each 2 to 8; or
(3) groups of formula

$$-(CH_2)_n(NH)(CO)(CH(NH(CO)CH_3))(CH_2)SS(CH_2)_pNH(PO_2)O\text{-}(Oligo)$$

and

$$-(CH_2)_n(NH)(CO)(CH(NH(CO)CH_3))(CH_2)SH;$$

or
(B) a cross-linked dextran macroporous material derivatized at hydroxyl oxygens, which are on carbons of the sugar moieties which have at least one neighboring carbon with an underivatized hydroxyl, with
(1) groups of formula

$-C(=NH)NH(CH_2)_q(NH)(CO)(CH_2)_cR_1$ and
$-C(=NH)NH(CH_2)_q(NH)(CO)(CH_2)_pNH(PO_2)O\text{-}(Oligo)$ with

substantially no hydroxyl oxygen derivatized with a group terminated with an amino group, or
(2) a group of formula

$-C(=NH)NH(CH_2)_p(NH)(PO_2)O\text{-}(Oligo)$, or

(3) groups of formula

$-C(=NH)NH(CH_2)_qCO_2H$ and

(i) $L_2\text{-}NH(PO_2)O\text{-}(Oligo)$,or

(ii) $L_2\text{-}SS(CH_2)_rNH(PO_2)O\text{-}(Oligo)$

wherein c,q, p and r are the same or different and q is 2 to 10 and wherein $L_2$ is

$-C(=NH)NH(CH_2)_q(CO)(NH)(CH_2)_p\text{-}$,

wherein the $-C(=NH)$ group is bonded to a hydroxyl oxygen of the dextran; or
(4) groups of formula

$-C(=NH)NH(CH_2)_nSH$ and
$-C(=NH)NH(CH_2)_nSS(CH_2)_pNH(PO_2)O\text{-}(Oligo)$;

and
(C) polystyrene, which may optionally be cross-linked with a cross-linking agent such as divinylbenzene, and which is derivatized at phenyl groups with
(1) groups of formula

$-(CH_2)_sCO_2H$ and

(i) $L_3\text{-}NH(PO_2)O\text{-}(Oligo)$, or

(ii) $L_3$-$SS(CH_2)_nNH(PO_2)O$-(Oligo),

wherein $L_3$ is $-(CH_2)_s(CO)(NH)(CH_2)_p-$,
wherein the $(CH_2)_s$ group is bonded to a phenyl group of the polystyrene and wherein s, p and n are the same or different and s is 2 to 10; or
(2) groups of formula

$-(CH_2)_s(CO)(NH)(CH_2)_p(NH)(CO)(CH_2)_cR_1$

and

$-(CH_2)_s(CO)(NH)(CH_2)_p(NH)(PO_2)O$-(Oligo),

with substantially no phenyl group derivatized with a group terminated with an amino group; or
(3) groups of formula

$-(CH_2)_s(CO)(NH)(CH_2)_pSH$, and
$-(CH_2)_s(CO)(NH)(CH_2)_pSS(CH_2)_nNH(PO_2)O$-(Oligo).

**11.** A solid support according to Claim 10 wherein the oligonucleotide is bound through the 5'-oxygen of the 5'-nucleoside and wherein the support is selected from:
(A) a controlled pore glass, in the form of beads of diameter between about 100 to 200 µm and pore diameter between about 0.04 and 0.06 µm (400-600 Angstroms), and derivatized at silicons with groups of formula

$-(CH_2)_a(NH)(CO)(CH_2)_bCH_3$ and
$-(CH_2)_a(NH)(PO_2)O$-(Oligo), with

substantially no silicon derivatized with a group terminated with an amino group, wherein a is 3 or 6 and b is 0, 1 or 2; and
(B) a macroporous material, made by cross-linking allyldextran with N,N'-methylenebisacrylamide, in the form of beads with a wet diameter between about 30 and about 120µm (microns) and an exclusion volume for dextrans between about $10^6$ daltons and about $10^8$ daltons, and derivatized, at hydroxyl oxygens, which are on carbons of the sugar moieties which have at least one neighboring carbon with an underivatized hydroxyl, with groups of formula

$-C(=NH)NH(CH_2)_6NH(CO)(CH_2)_bCH_3$ and
$-C(=NH)NH(CH_2)_6NH(CO)(CH_2)_6NH(PO_2)O$-(Oligo)

with substantially no hydroxyl oxygen derivatized with a group terminated with an amino group, or a group of formula

$-C(=NH)NH(CH_2)_6NH(PO_2)O$-(Oligo).

**12.** A solid support according to Claim 10 wherein the oligonucleotide is bound to the solid support through the 5'-oxygen of the 5'-nucleoside and wherein the solid support is selected from:
(A) a controlled pore glass, in the form of beads of diameter about 100 and about 200 µm (microns) and pore diameter between about 0.04 and 0.06 µm (400-600) Angstroms and derivatized at silicons with groups of formula

$-(CH_2)_3NH(CO)(CH_2)_2CO_2H$ and
$-(CH_2)_3NH(CO)(CH_2)_2(CO)(NH)(CH_2)_6NH(PO_2)O$-(Oligo);

and
(B) a macroporous material, made by cross-linking allyldextran with N,N'-methylenebisacrylamide, in the form of beads with a wet diameter between about 30 and about 120µm (microns) and an exclusion volume for dextrans between about $10^6$ daltons and about $10^8$ daltons, and derivatized at hydroxyl oxygens, which are on carbons of the sugar moieties which have at least one neighboring

carbon with an underivatized hydroxyl, with groups of formula

$-C(=NH)NH(CH_2)_5CO_2H$ and
$-C(=NH)NH(CH_2)_5(CO)(NH)(CH_2)_6NH(PO_2)O$-(Oligo)

and
(C) 0% to about 16% divinylbenzene-crosslinked polystyrene beads, of between about 60 and 500 mesh size, derivatized at polystyrene phenyls with groups of formula

$-(CH_2)_4CO_2H$ and
$-(CH_2)_4(CO)(NH)(CH_2)_6(NH)(PO_2)O$-(Oligo).

**13.** A solid support according to Claim 11 or 12 wherein the oligonucleotide has 25-50 bases.

**14.** A solid support according to Claim 13 wherein the oligonucleotide is a DNA or RNA which has a sequence which is complementary to the sequence of a segment of the genome of HIV-1.

**15.** A test kit for carrying out the method of any one of Claims 1 to 9 for detecting, in a nucleic acid probe sandwich assay, a single-stranded nucleic acid analyte, which kit comprises (A) a solid support according to any of Claims 12 to 16, wherein (Oligo) has a sequence complementary to the sequence of a first segment of said analyte and (B) a second probe, which is a single-stranded oligonucleotide with a sequence complementary to the sequence of a second segment of said analyte and is labeled for detection, provided that the sequence of the second probe is non-complementary to that of the first probe and the second segment does not overlap the first segment.

**16.** An RNA or a DNA for use in the method of any one of Claims 1 to 9 with a sequence selected from the group consisting of:

**5'-CAAAAACTATTCTTAAACCTACCAAGCCTC-3'**
**5'-TATTACATTTTAGAATCGCAAAACCAGCC-3'**
**5'-TAGGTTTCCCTGAAACATACATATGGTGT-3'**
**5'-TGGTCTGCTAGTTCAGGGTCTACTTGTGTGC-3'**
**5'-CACCTAGGGCTAACTATGTGTCCTAATAAGG-3'**
**5'-TTTCGTAACACTAGGCAAAGGTGGCTTTATC-3'**
**5'-TGGTCTTCTGGGGCTTGTTCCATCTATCCTC-3'**
**5'-AGGGAAAATGTCTAACAGCTTCATTCTTAAC-3'**

5'-AAATGGATAAACAGCAGTTGTTGCAGAATTC-3'
5'-TCGAGTAACGCCTATTCTGCTATGTCGACAC-3'
5'-CTGTGTAATGACTGAGGTGTTACAACTTGT-3'
5'-TCTAATTACTACCTCTTCTTCTGCTAGACT-3'
5'-AATATGTTGTTATTACCAATCTAGCAT-3,

and any sequence complementary to any of the above-specified, provided that, in an RNA, a T in the above-specified sequences represents a uridine.

**Patentansprüche**

1. Verfahren zum Nachweis eines einzelsträngigen Nukleinsäureanalyten in einer Probe unter Verwendung:
   (a) eines festen Trägers, der eine erste Sonde enthält, die ein einzelsträngiges Oligonukleotid zwischen 15 und 100 Basen in einer Sequenz ist, die komplementär zu der Sequenz eines ersten Segments des Analyten ist, wobei ein erheblicher Anteil der ersten Sonde, die Teil des festen Trägers ist, kovalent und nur durch einen Linker, der entweder mit dem 5'-Sauerstoff des 5'-terminalen Nukleosids oder dem 3'-Sauerstoff des 3'-terminalen Nukleosids verknüpft ist, an den Träger gebunden ist und
   (b) einer zweiten Sonde, die ein einzelsträngiges Oligonukleotid mit einer Sequenz ist, die komplementär zu der Sequenz eines zweiten Segments des Analyten ist und zum Nachweis markiert ist, vorausgesetzt, daß die Sequenz der zweiten Sonde nicht komplementär zu der der ersten Sonde ist und das zweite Segment nicht mit dem ersten Segment überlappt, wobei das Verfahren umfaßt:
   (1) Inkubieren des festen Trägers unter Vorhybridisierungsbedingungen mit einer Vorhybridisierungslösung, die (i) eine nicht-homologe Nukleinsäure oder (ii) Dextransulfat in einer Konzentration zwischen etwa 5 % (Gew./Vol.) und 20 % (Gew./Vol.) oder sowohl (i) als auch (ii) umfaßt,
   (2) Inkubieren des gemäß Schritt (1) vorhybridisierten festen Trägers unter Hybridisierungsbedingungen mit einer Hybridisierungslösung, die zwischen etwa 5 % (Gew./Vol.) und 20 % (Gew./Vol.) Dextransulfat, Nukleinsäure aus der zu testenden Probe einschließlich des Analyten, sofern er in der Probe vorhanden ist, und die zweite Sonde in molarem Überschuß relativ zur Menge des Analyten in der Hybridisierungslösung enthält,
   (3) Waschen des festen Trägers nach Schritt (2), um eine zweite Sonde davon zu entfernen, die nicht stabil mit dem Analyten hybridisiert hat und
   (4) Bestimmen nach Schritt (3), ob ein Signal aufgrund der nachweisbaren Markierung an der mit dem Analyten hybridisierten zweiten Sonde mit dem festen Träger assoziiert ist.

2. Verfahren nach Anspruch 1, worin der feste Träger, an den die erste Sonde gebunden ist, ausgewählt ist aus:
   (A) porösem Silikatglas, das an Siliciumatomen derivatisiert ist mit
   (1) Gruppen der Formel

   $-(CH_2)_n(NH)(CO)(CH_2)_cR_1$ und
   $-(CH_2)_n(NH)(PO_2)O\text{-(Oligo)}$,

   wobei im wesentlichen kein Silicium mit einer Gruppe derivatisiert ist, die mit einer Aminogruppe endet, worin c 0 bis 5 ist, n 2 bis 8 ist, $R_1$ Methyl oder Benzyl ist, -O-(Oligo) die Oligonukleotidsonde ist und das mit (Oligo) verknüpfte Sauerstoffatom der 5'-Sauerstoff des 5'-Nukleosids oder der 3'-Sauerstoff des 3'-Nukleosids der Sonde ist, oder
   (2) Gruppen der Formel

   $-L_1-CO_2H$ und

   (i) $-L_1-(CO)(NH)(CH_2)_pNH(PO_2)O\text{-(Oligo)}$ oder

   (ii) $-L_1-(CO)(NH)(CH_2)_pSS(CH_2)_rNH(PO_2)O\text{-(Oligo)}$,

   worin $-L_1$ $-(CH_2)_n(NH)(CO)(CH_2)_m-$ ist, worin die Gruppe $-(CH_2)_n$ mit einem Siliciumatom verknüpft ist und worin m, n, p und r gleich oder verschieden und jeweils 2 bis 8 sind, oder
   (3) Gruppen der Formel

$$-(CH_2)_n(NH)(CO)(\underset{}{\overset{NH(CO)CH_3}{\overset{|}{C}H}})(CH_2)SS(CH_2)_pNH(PO_2)O\text{-(Oligo)}$$

   und

$$-(CH_2)_n(NH)(CO)(\overset{NH(CO)CH_3}{\overset{|}{CH}})(CH_2)SH,$$

oder

(B) einem quervernetzten, makroporigen Dextranmaterial, das an Hydroxyl-Sauerstoffatomen, die sich an Kohlenstoffatomen der Zuckergruppierungen befinden, die mindestens ein benachbartes Kohlenstoffatom mit einem nichtderivatisierten Hydroxyl aufweisen, derivatisiert ist mit

(1) Gruppen der Formel

$-C(=NH)NH(CH_2)_q(NH)(CO)(CH_2)_cR_1$ und
$-C(=NH)NH(CH_2)q(NH)(CO)(CH_2)_pNH(PO_2)O$-(Oligo),

wobei im wesentlichen kein Hydroxyl-Sauerstoffatom mit einer Gruppe derivatisiert ist, die mit einer Aminogruppe endet, oder

(2) einer Gruppe der Formel

$-C(=NH)NH(CH_2)_p(NH)(PO_2)O$-(Oligo) oder

(3) Gruppen der Formel

$-C(=NH)NH(CH_2)_qCO_2H$ und

( i) $L_2$-$NH(PO_2)O$-(Oligo), oder

(ii) $L_2$-$SS(CH_2)_rNH(PO_2)O$-(Oligo),

worin c, q, p und r gleich oder verschieden sind und q 2 bis 10 ist und worin $L_2$

$-C(=NH)NH(CH_2)_q(CO)(NH)(CH_2)_p-$

ist, worin die -C(=NH)-Gruppe mit einem Hydroxyl-Sauerstoff des Dextrans verknüpft ist oder

(4) Gruppen der Formel

$-C(=NH)NH(CH_2)_nSH$ und
$-C(=NH)NH(CH_2)_nSS(CH_2)_pNH(PO_2)O$-(Oligo)

und

(C) Polystyrol, welches gegebenenfalls mit einem Quervernetzungsmittel wie etwa Divinylbenzol quervernetzt sein kann, und das an Phenylgruppen derivatisiert ist mit

(1) Gruppen der Formel

$-(CH_2)_sCO_2H$ und

(i) $L_3$-$NH(PO_2)O$-(Oligo) oder

(ii) $L_3$-$SS(CH_2)_nNH(PO_2)O$-(Oligo),

worin $L_3$ $-(CH_2)_s(CO)(NH)(CH_2)_p-$ ist, worin die $(CH_2)_s$-Gruppe mit einer Phenylgruppe des Polystyrols verknüpft ist und worin s, p und n gleich oder verschieden sind und s 2 bis 10 ist, oder

(2) Gruppen der Formel

$-(CH_2)_s(CO)(NH)(CH_2)_p(NH)(CO)(CH_2)_cR_1$ und
$-(CH_2)_s(CO)(NH)(CH_2)_p(NH)(PO_2)O$-(Oligo),

wobei im wesentlichen keine Phenylgruppe mit einer Gruppe derivatisiert ist, die mit einer Aminogruppe endet, oder
(3) Gruppen der Formel

$-(CH_2)_s(CO)(NH)(CH_2)_pSH$ und
$-(CH_2)_s(CO)(NH)(CH_2)_pSS(CH_2)_nNH(PO_2)O$-(Oligo).

**3.** Verfahren nach Anspruch 2, worin die erste Sonde durch den 5'-Sauerstoff des 5'-Nukleosids an den festen Träger gebunden ist und worin der feste Träger mit der daran gebundenen ersten Sonde ausgewählt ist aus:
(A) einem Glas mit kontrollierten Poren in Form von Körnern eines Durchmessers zwischen etwa 100 und etwa 200 µm (Micron) und einem Porendurchmesser zwischen etwa 0,04 µm und 0,06 µm (400 bis 600 Angström) und das an Siliciumatomen derivatisiert ist mit Gruppen der Formel

$-(CH_2)_a(NH)(CO)(CH_2)_bCH_3$ und
$-(CH_2)_a(NH)(PO_2)O$-(Oligo)

wobei im wesentlichen kein Siliciumatom mit einer Gruppe derivatisiert ist, die mit einer Aminogruppe endet, worin a 3 oder 6 ist und b 0, 1 oder 2 ist und
(B) einem makroporigen Material, das durch Quervernetzung von Allyldextran mit N,N'-Methylenbisacrylamid hergestellt wurde, in Form von Körnern mit einem Naßdurchmesser zwischen etwa 30 und etwa 120 µm (Micron) und einem Ausschlußvolumen für Dextrane zwischen etwa $10^6$ Dalton und etwa $10^8$ Dalton und das an Hydroxyl-Sauerstoffatomen, die sich an Kohlenstoffatomen der Zuckergruppierungen befinden, die mindestens ein benachbartes Kohlenstoffatom mit einem nicht-derivatisierten Hydroxyl enthalten, derivatisiert ist mit Gruppen der Formel

$-C(=NH)NH(CH_2)_6NH(CO)(CH_2)_bCH_3$ und
$-C(=NH)NH(CH_2)_6NH(CO)(CH_2)_pNH(PO_2)O$-(Oligo)

oder

$-C(=NH)NH(CH_2)_6NH(PO_2)O$-(Oligo),

wobei im wesentlichen kein Hydroxyl-Sauerstoffatom mit einer Gruppe derivatisiert ist, die mit einer Aminogruppe endet.

**4.** Verfahren nach Anspruch 2, worin die erste Sonde durch den 5'-Sauerstoff des 5'-Nukleosids an den festen Träger gebunden ist und worin der feste Träger mit der daran gebundenen ersten Sonde ausgewählt ist aus:
(A) einem Glas mit kontrollierten Poren in Form von Körnern eines Durchmessers zwischen etwa 100 und etwa 200 µm (Micron) und einem Porendurchmesser zwischen etwa 0,04 µm und 0,06 µm (400 bis 600 Angström) und das an Siliciumatomen derivatisiert ist mit Gruppen der Formel

$-(CH_2)_3NH(CO)(CH_2)_2CO_2H$ und
$-(CH_2)_3NH(CO)(CH_2)_2(CO)(NH)(CH_2)_6NH(PO_2)O$-(Oligo)

und
(B) einem makroporigen Material, das durch Quervernetzung von Allyldextran mit N,N'-Methylenbisacrylamid hergestellt wurde, in Form von Körnern mit einem Naßdurchmesser zwischen etwa 30 und etwa 120 µm (Micron) und einem Ausschlußvolumen für Dextrane zwischen etwa $10^6$ Dalton und etwa $10^8$ Dalton und das an Hydroxyl-Sauerstoffatomen, die sich an Kohlenstoffatomen der Zuckergruppierungen befinden, die mindestens ein benachbartes Kohlenstoffatom mit einem nicht-derivatisierten Hydroxyl enthalten, derivatisiert ist mit Gruppen der Formel

$-C(=NH)NH(CH_2)_5CO_2H$ und
$-C(=NH)NH(CH_2)_5(CO)(NH)(CH_2)_6NH(PO_2)O$-(Oligo)

und
(C) mit 0 % bis etwa 16 % Divinylbenzol quervernetzten Polystyrolkörnern von einer Größe zwischen etwa 60 und 500 mesh, die an den Polystyrol-Phenylgruppen derivatisiert sind mit Gruppen der Formel

$-(CH_2)_4CO_2H$ und
$-(CH_2)_4(CO)(NH)(CH_2)_6(NH)(PO_2)O$-(Oligo).

**5.** Verfahren nach Anspruch 3 oder 4, worin sowohl die erste Sonde als auch die zweite Sonde 25 bis 50 Basen aufweisen und die zweite Sonde radioaktiv markiert ist.

**6.** Verfahren nach Anspruch 5, worin die radioaktive Markierung auf der zweiten Sonde ein $^{32}P$ einer an den 5'-Kohlenstoff des 5'-Nukleosids gebundenen Phosphatgruppe ist.

**7.** Verfahren nach Anspruch 6, worin sowohl die Vorhybridisierungslösung als auch die Hybridisierungslösung zwischen etwa 8 % (Gew./Vol.) und etwa 12 % (Gew./Vol.) Dextransulfat umfassen.

**8.** Verfahren nach Anspruch 7, worin vor Schritt (2) gemäß Anspruch 1 Nukleinsäure aus der zu testenden Probe unter Hybridisierungsbedingungen in einer ersten Hybridisierungslösung inkubiert wird, die einen molaren Überschuß an zweiter Sonde bezüglich der Konzentration des Analyten in der Hybridisierungslösung aufweist, bis ein nennenswerter Anteil eines vorhandenen Analyten mit der zweiten Sonde hybridisiert hat, und dann Schritt (2) gemäß Anspruch 1 durch Vereinigung des gemäß Schritt (1) von Anspruch 1 vorhybridisierten festen Trägers mit der ersten Hybridisierungslösung und Eintauchen in eine zweite Hybridisierungslösung gestartet wird.

**9.** Verfahren nach einem der Ansprüche 5 bis 8, worin der Analyt das Genom eines HIV-1-Virus oder ein Fragment davon ist, das Segmente mit Sequenzen umfaßt, die komplementär zur ersten Sonde und zur zweiten Sonde sind.

**10.** Fester Träger zur Anwendung bei dem Verfahren nach einem der Ansprüche 1 bis 9 mit einer daran gebundenen einzelsträngigen Oligonukleotidsonde von 15 bis 100 Basen und ausgewählt aus
(A) porösem Silikatglas, das an Siliciumatomen derivatisiert ist mit
(1) Gruppen der Formel

$-(CH_2)_n(NH)(CO)(CH_2)_cR_1$ und
$-(CH_2)_n(NH)(PO_2)O$-(Oligo),

wobei im wesentlichen kein Silicium mit einer Gruppe derivatisiert ist, die mit einer Aminogruppe endet, worin c 0 bis 5 ist, n 2 bis 8 ist, $R_1$ Methyl oder Benzyl ist, -O-(Oligo) die Oligonukleotidsonde ist und das mit (Oligo) verknüpfte Sauerstoffatom der 5'-Sauerstoff des 5'-Nukleosids oder der 3'-Sauerstoff des 3'-Nukleosids der Sonde ist, oder
(2) Gruppen der Formel

$-L_1-CO_2H$ und

(i) $-L_1-(CO)(NH)(CH_2)_pNH(PO_2)O$-(Oligo) oder

(ii)$-L_1-(CO)(NH)(CH_2)_pSS(CH_2)_rNH(PO_2)O$-(Oligo),

worin $-L_1-(CH_2)_n(NH)(CO)(CH_2)m$- ist, worin die Gruppe $-(CH_2)_n$ mit einem Siliciumatom verknüpft ist und worin m, n, p und r gleich oder verschieden und jeweils 2 bis 8 sind, oder
(3) Gruppen der Formel

$$\begin{array}{c} \qquad\qquad\qquad\qquad NH(CO)CH_3 \\ \qquad\qquad\qquad\qquad | \\ -(CH_2)_n(NH)(CO)(CH)(CH_2)SS(CH_2)_pNH(PO_2)O\text{-(Oligo)} \end{array}$$

und

$$\begin{array}{c} \qquad\qquad\qquad\qquad\qquad\quad NH(CO)CH_3 \\ -(CH_2)_n(NH)(CO)(CH)(CH_2)SH, \end{array}$$

oder

(B) einem quervernetzten, makroporigen Dextranmaterial, das an Hydroxyl-Sauerstoffatomen, die sich an Kohlenstoffatomen der Zuckergruppierungen befinden, die mindestens ein benachbartes Kohlenstoffatom mit einem nichtderivatisierten Hydroxyl aufweisen, derivatisiert ist mit

(1) Gruppen der Formel

$-C(=NH)NH(CH_2)_q(NH)(CO)(CH_2)_cR_1$ und
$-C(=NH)NH(CH_2)_q(NH)(CO)(CH_2)_pNH(PO_2)O$-(Oligo),

wobei im wesentlichen kein Hydroxyl-Sauerstoffatom mit einer Gruppe derivatisiert ist, die mit einer Aminogruppe endet, oder

(2) einer Gruppe der Formel

$-C(=NH)NH(CH_2)_p(NH)(PO_2)O$-(Oligo) oder

(3) Gruppen der Formel

$-C(=NH)NH(CH_2)_qCO_2H$ und

(i) $L_2$-$NH(PO_2)O$-(Oligo), oder

(ii) $L_2$-$SS(CH_2)_rNH(PO_2)O$-(Oligo),

worin c, q, p und r gleich oder verschieden sind und q 2 bis 10 ist und worin $L_2$

$-C(=NH)NH(CH_2)_q(CO)(NH)(CH_2)_p-$

ist, worin die -C(=NH)-Gruppe mit einem Hydroxyl-Sauerstoff des Dextrans verknüpft ist oder

(4) Gruppen der Formel

$-C(=NH)NH(CH_2)_nSH$ und
$-C(=NH)NH(CH_2)_nSS(CH_2)_pNH(PO_2)O$-(Oligo)

und

(C) Polystyrol, welches gegebenenfalls mit einem Quervernetzungsmittel wie etwa Divinylbenzol quervernetzt sein kann, und das an Phenylgruppen derivatisiert ist mit

(1) Gruppen der Formel

$-(CH_2)_sCO_2H$ und

(i) $L_3$-$NH(PO_2)O$-(Oligo) oder

(ii) $L_3$-$SS(CH_2)_nNH(PO_2)O$-(Oligo),

worin $L_3$ $-(CH_2)_s(CO)(NH)(CH_2)_p-$ ist, worin die $(CH_2)_s$-Gruppe mit einer Phenylgruppe des Polystyrols verknüpft ist und worin s, p und n gleich oder verschieden sind und s 2 bis 10 ist, oder

(2) Gruppen der Formel

$-(CH_2)_s(CO)(NH)(CH_2)_p(NH)(CO)(CH_2)_cR_1$ und
$-(CH_2)_s(CO)(NH)(CH_2)_p(NH)(PO_2)O$-(Oligo),

wobei im wesentlichen keine Phenylgruppe mit einer Gruppe derivatisiert ist, die mit einer Aminogruppe endet, oder
(3) Gruppen der Formel

$-(CH_2)_s(CO)(NH)(CH_2)_pSH$ und
$-(CH_2)_s(CO)(NH)(CH_2)_pSS(CH_2)_nNH(PO_2)O$-(Oligo).

**11.** Fester Träger nach Anspruch 10, worin das Oligonukleotid durch den 5'-Sauerstoff des 5'-Nukleosids gebunden ist und worin der Träger ausgewählt ist aus:
(A) einem Glas mit kontrollierten Poren in Form von Körnern eines Durchmessers zwischen etwa 100 und 200 $\mu$m (Micron) und einem Porendurchmesser zwischen etwa 0,04 und 0,06 $\mu$m (400 bis 600 Angström) und an Siliciumatomen derivatisiert ist mit Gruppen der Formel

$-(CH_2)_a(NH)(CO)(CH_2)_bCH_3$ und
$-(CH_2)_a(NH)(PO_2)O$-(Oligo)

wobei im wesentlichen kein Siliciumatom mit einer Gruppe derivatisiert ist, die mit einer Aminogruppe endet, worin a 3 oder 6 ist und b 0, 1 oder 2 ist und
(B) einem makroporigen Material, das durch Quervernetzung von Allyldextran mit N,N'-Methylenbisacrylamid hergestellt wurde, in Form von Körnern mit einem Naßdurchmesser zwischen etwa 30 und etwa 120 $\mu$m (Micron) und einem Ausschlußvolumen für Dextrane zwischen etwa $10^6$ Dalton und etwa $10^8$ Dalton und das an Hydroxyl-Sauerstoffatomen, die sich an Kohlenstoffatomen der Zuckergruppen befinden, die mindestens ein benachbartes Kohlenstoffatom mit einem nicht-derivatisierten Hydroxyl enthalten, derivatisiert ist mit Gruppen der Formel

$-C(=NH)NH(CH_2)_6NH(CO)(CH_2)_bCH_3$ und
$-C(=NH)NH(CH_2)_6NH(CO)(CH_2)_6NH(PO_2)O$-(Oligo),

wobei im wesentlichen kein Hydroxyl-Sauerstoffatom mit einer Gruppe derivatisiert ist, die mit einer Aminogruppe endet, oder mit einer Gruppe der Formel

$-C(=NH)NH(CH_2)_6NH(PO_2)O$-(Oligo).

**12.** Fester Träger nach Anspruch 10, worin das Oligonukleotid durch den 5'-Sauerstoff des 5'-Nukleosids an den festen Träger gebunden ist und worin der feste Träger ausgewählt ist aus:
(A) einem Glas mit kontrollierten Poren in Form von Körnern eines Durchmessers zwischen etwa 100 und etwa 200 $\mu$m (Micron) und einem Porendurchmesser zwischen etwa 0,04 und 0,06 $\mu$m (400 bis 600 Angström) und an Siliciumatomen derivatisiert ist mit Gruppen der Formel

$-(CH_2)_3NH(CO)(CH_2)_2CO_2H$ und
$-(CH_2)_3NH(CO)(CH_2)_2(CO)(NH)(CH_2)_6NH(PO_2)O$-(Oligo)

und
(B) einem makroporigen Material, das durch Quervernetzung von Allyldextran mit N,N'-Methylenbisacrylamid hergestellt wurde, in Form von Körnern mit einem Naßdurchmesser zwischen etwa 30 und etwa 120 $\mu$m (Micron) und einem Ausschlußvolumen für Dextrane zwischen etwa $10^6$ Dalton und etwa $10^8$ Dalton und das an Hydroxyl-Sauerstoffatomen, die sich an Kohlenstoffatomen der Zuckergruppen befinden, die mindestens ein benachbartes Kohlenstoffatom mit einem nicht-derivatisierten Hydroxyl enthalten, derivatisiert ist mit Gruppen der Formel

$-C(=NH)NH(CH_2)_5CO_2H$ und
$-C(=NH)NH(CH_2)_5(CO)(NH)(CH_2)_6NH(PO_2)O$-(Oligo)

und

(C) mit 0 % bis etwa 16 % Divinylbenzol quervernetzten Polystyrolkörnern von einer Größe zwischen etwa 60 und 500 mesh, die an den Polystyrol-Phenylgruppen derivatisiert sind mit Gruppen der Formel

$-(CH_2)_4CO_2H$ und
$-(CH_2)_4(CO)(NH)(CH_2)_6(NH)(PO_2)O$-(Oligo).

**13.** Fester Träger nach Anspruch 11 oder 12, worin das Oligonukleotid 25 bis 50 Basen besitzt.

**14.** Fester Träger nach Anspruch 13, worin das Oligonukleotid eine DNA oder RNA ist, die eine Sequenz aufweist, die komplementär zur Sequenz eines Segments des Genoms von HIV-1 ist.

**15.** Testkit zur Durchführung des Verfahrens nach einem der Ansprüche 1 bis 9 zum Nachweis eines einzelsträngigen Nukleinsäureanalyten in einem Nukleinsäuresonden-Sandwichassay, wobei der Kit umfaßt (A) einen festen Träger nach einem der Ansprüche 12 bis 16, worin (Oligo) eine Sequenz besitzt, die komplementär zur Sequenz eines ersten Segements des Analyten ist und (B) eine zweite Sonde, die ein einzelsträngiges Oligonukleotid mit einer Sequenz ist, die komplementär zur Sequenz eines zweiten Segments des Analyten ist und zum Nachweis markiert ist, vorausgesetzt, daß die Sequenz der ersten Sonde nicht komplementär zu der der zweiten Sonde ist und daß das zweite Segment nicht mit dem ersten Segment überlappt.

**16.** RNA oder DNA zur Anwendung im Verfahren nach einem der Ansprüche 1 bis 9 mit einer Sequenz, die aus der Gruppe ausgewählt ist, bestehend aus:

5'-CAAAAACTATTCTTAAACCTACCAAGCCTC-3'
5'-TATTACATTTTAGAATCGCAAAACCAGCC-3'
5'-TAGGTTTCCCTGAAACATACATATGGTGT-3'
5'-TGGTCTGCTAGTTCAGGGTCTACTTGTGTGC-3'
5'-CACCTAGGGCTAACTATGTGTCCTAATAAGG-3'
5'-TTTCGTAACACTAGGCAAAGGTGGCTTTATC-3'
5'-TGGTCTTCTGGGGCTTGTTCCATCTATCCTC-3'
5'-AGGGAAAATGTCTAACAGCTTCATTCTTAAC-3'
5'-AAATGGATAAACAGCAGTTGTTGCAGAATTC-3'
5'-TCGAGTAACGCCTATTCTGCTATGTCGACAC-3'
5'-CTGTGTAATGACTGAGGTGTTACAACTTGT-3'
5'-TCTAATTACTACCTCTTCTTCTGCTAGACT-3'
5'-AATATGTTGTTATTACCAATCTAGCAT-3,

und jeder Sequenz, die komplementär zu einer der oben spezifizierten ist,
unter der Voraussetzung, daß in einer RNA ein T in den oben spezifizierten Sequenzen ein Uridin bedeutet.

## Revendications

**1.** Procédé de détection d'un analyte d'acide nucléique simple brin dans un échantillon en utilisant :
(a) un support solide comprenant une première sonde, consistant en un oligonucléotide simple brin de 15 à 100 bases ordonnées suivant une séquence complémentaire d'un premier segment du dit analyte, une fraction importante de la dite première sonde qui fait partie du dit support solide étant liée au dit support par liaison covalente et uniquement par l'intermédiaire d'un liant fixé soit sur l'oxygène 5' du nucléoside 5' terminal, soit sur l'oxygène 3' du nucléoside 3' terminal, et

(b) une deuxième sonde, qui est un oligonucléotide simple brin comportant une séquence complémentaire de la séquence d'un second segment du dit analyte et qui est marquée pour la détection, étant entendu que la séquence de la seconde sonde n'est pas complémentaire de celle de la première et que le second segment ne recouvre pas le premier segment, ce procédé comprenant

(1) l'incubation du support solide en conditions de préhybridation avec une solution de préhybridation comprenant (i) un acide nucléique non homologue, ou (ii) du sulfate de dextran à une concentration comprise environ entre 5 % (p/v) et 20 % (p/v), ou bien (i) et (ii) ensemble ;

(2) l'incubation en conditions d'hybridation du support solide, préhybridé selon l'étape (1), avec une solution d'hybridation comprenant du sulfate de dextran à une concentration comprise environ entre 5 % (p/v) et 20 % (p/v) ; de l'acide nucléique de l'échantillon en cours d'analyse, comprenant l'analyte si celui-ci est présent dans le dit échantillon ; et la deuxième sonde, en excès molaire par rapport à la quantité d'analyte contenue dans la solution d'hybridation ;

(3) après l'étape (2), rinçage du support solide pour éliminer de celui-ci la deuxième sonde ne s'étant pas hybridée de façon stable à l'analyte; et

(4) après l'étape (3), détermination de l'association éventuelle au dit support solide d'un signal dû au marqueur détectable sur la deuxième sonde hybridée à l'analyte.

**2.** Procédé selon la revendication 1 dans lequel le support solide sur lequel est fixée la première sonde est choisi parmi :

(A) un verte de silicate poreux dérivé sur les silicones par

(1) des groupements de formule

$-(CH_2)_n(NH)(CO)(CH_2)_cR_1$ et
$-(CH_2)_n(NH)(PO_2)O\text{-}(Oligo)$,

avec substantiellement aucun silicone dérivé par un groupement se terminant par un groupement amine, dans lesquels c est compris entre 0 et 5, n est compris entre 2 et 8, $R_1$ est un méthyle ou un benzyle, -O-(Oligo) est la sonde d'oligonucléotide, et l'atome d'oxygène fixé sur (Oligo) est l'oxygène 5' du nucléoside 5' ou l'oxygène 3' du nucléoside 3' de la sonde ; ou

(2) des groupements de formule

$-L_1\text{-}CO_2H$ et

(i) $-L_1\text{-}(CO)(NH)(CH_2)_pNH(PO_2)O\text{-}(Oligo)$, ou

(ii) $-L_1\text{-}(CO)(NH)(CH_2)_pSS(CH_2)_rNH(PO_2)O\text{-}(Oligo)$,

dans lesquels $-L_1-$ est $-(CH_2)n(NH)(CO)(CH_2)m-$, dans lequel le groupement $-(CH_2)n$ est lié à un atome de silicone ; et dans lesquels m, n, p et r sont égaux ou différents et sont chacun compris entre 2 et 8 ; ou

(3) des groupements de formule

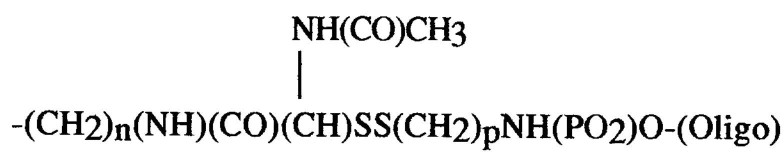

et

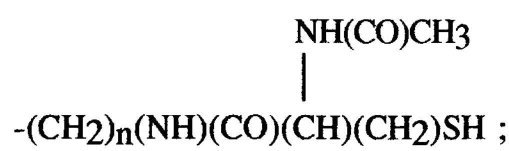

ou

(B) un matériau macroporeux de dextran réticulé dérivé sur les oxygènes des hydroxyles, eux-mêmes portés par les carbones des fragments sucres comportant au moins un carbone avec un hydroxyle non dérivé à proximité, avec

(1) des groupements de formule

$-C(=NH)NH(CH_2)_q(NH)(CO)(CH_2)_cR_1$ et
$-C(=NH)NH(CH_2)_q(NH)(CO)(CH_2)_pNH(PO_2)O$-(Oligo)

avec substantiellement aucun oxygène d'hydroxyle dérivé par un groupement se terminant par un groupement amine, ou

(2) un groupement de formule

$-C(=NH)NH(CH_2)_p(NH)(PO_2)O$-(Oligo), ou

(3) des groupements de formule

$-C(=NH)NH(CH_2)_qCO_2H$ et

(i) L2-$NH(PO_2)O$-(Oligo), Ou

(ii) $L_2$-$SS(CH_2)_rNH(PO_2)O$-(Oligo) dans lesquels c, q, p et r sont égaux ou différents et q est compris entre 2 et 10 et dans lesquels $L_2$ est
$-C(=NH)NH(CH_2)_q(CO)(NH)(CH_2)p$-, dans lequel le groupement $-C(=NH)$ est lié à l'oxygène d'un hydroxyle du dextran ; ou

(4) des groupements de formule

$-C(=NH)NH(CH_2)nSH$ et
$-C(=NH)NH(CH_2)_nSS(CH_2)_pNH(PO_2)O$-(Oligo) ; et

(C) du polystyrène éventuellement réticulé par un agent réticulant tel que le divinylbenzène, et qui est dérivé sur les groupements phényles par

(1) des groupements de formule

$-(CH_2)_sCO_2H$ et

( i) $L_3$-$NH(PO_2)O$-(Oligo), Ou

(ii) $L_3$-$SS(CH_2)_nNH(PO_2)O$-(Oligo), dans lesquels $L_3$ est $-(CH_2)_s(CO)(NH)(CH_2)p$-, dans lequel le groupement $(CH_2)_s$ est lié à un groupement phényle du polystyrène et dans lesquels s, p et n sont égaux ou différents et s est compris entre 2 et 10 ; ou

(2) des groupements de formule

$-(CH_2)_s(CO)(NH)(CH_2)_p(NH)(CO)(CH_2)_cR_1$ et
$-(CH_2)_s(CO)(NH)(CH_2)_p(NH)(PO_2)O$-(Oligo),

avec substantiellement aucun groupement phényle dérivé par un groupement terminé par un groupement amine; ou

(3) des groupements de formule

$-(CH_2)_s(CO)(NH)(CH_2)_pSH$, et
$-(CH_2)_s(CO)(NH)(CH_2)_pSS(CH_2)_nNH(PO_2)O$-(Oligo).

**3.** Procédé selon la revendication 2 dans lequel la première sonde est fixée sur le support solide par l'oxygène 5' du nucléoside 5' et dans lequel le support comprenant cette première sonde est choisi parmi :

(A) un verte à pores contrôlés, sous forme de billes de diamètre compris entre environ 100 et 200 µm (microns) et dont le diamètre des pores est compris entre environ 0,04 µm et 0,06 µm (400 à 600 Angstroms) et dérivé sur les silicones par des groupements de formule

$-(CH_2)_a(NH)(CO)(CH_2)_bCH_3$ et
$-(CH_2)_a(NH)(PO_2)O$-(Oligo)

avec substantiellement aucun silicone dérivé par un groupement terminé par un groupement amine, dans lesquels a est égal à 3 ou 6 et b est égal à 0, 1, ou 2 ; et

(B) un matériau macroporeux, obtenu par réticulation d'allyldextran par du N,N'-méthylènebisacrylamide, sous forme de billes dont le diamètre, à l'état humide, est compris entre environ 30 et 120 µm (microns) et le volume d'exclusion pour les dextrans est compris entre environ $10^6$ et $10^8$ daltons, et dérivé, sur les oxygènes des hydroxyles, eux-mêmes sur des carbones des fragments sucres comportant au moins un carbone portant un hydroxyle non dérivé à proximité, par des groupements de formule

$-C(=NH)NH(CH_2)_6NH(CO)(CH_2)_bCH_3$ et
$-C(=NH)NH(CH_2)_6NH(CO)(CH_2)_pNH(PO_2)O$-(Oligo), ou
$-C(=NH)NH(CH_2)_6NH(PO_2)O$-(Oligo)

avec substantiellement aucun oxygène d'hydroxyle dérivé par un groupement terminé par un groupement amine.

4. Procédé selon la revendication 2 dans lequel la première sonde est fixée sur le support solide par l'oxygène 5' du nucléoside 5' et dans lequel le support, avec cette première sonde liée, est choisi parmi :

(A) un verre à pores contrôlés, sous forme de billes de diamètre compris entre environ 100 et 200 µm (microns) et dont le diamètre des pores est compris entre environ 0,04 µm et 0,06 µm (400 à 600 Angstroms) et dérivé sur les silicones par des groupements de formule

$-(CH_2)_3(NH)(CO)(CH_2)_2CO_2H$ et
$-(CH_2)_aNH(CO)(CH_2)_2(CO)(NH)(CH_2)_6NH(PO_2)O$-(Oligo)

et

(B) un matériau macroporeux, obtenu par réticulation d'allyldextran par du N,N'-méthylènebisacrylamide, sous forme de billes dont le diamètre, à l'état humide, est compris entre environ 30 et 120 µm (microns) et le volume d'exclusion pour les dextrans est compris entre environ $10^6$ et $10^8$ daltons, et dérivé, sur les oxygènes des hydroxyles, eux-mêmes sur des carbones des fragments sucres comportant au moins un carbone portant un hydroxyle non dérivê à proximité, par des groupements de formule

$-C(=NH)NH(CH_2)_5CO_2H$ et
$-C(=NH)NH(CH_2)_5(CO)(NH)(CH_2)_6NH(PO_2)O$-(Oligo),

et

(C) 0 à environ 16 % de billes de polystyrène réticulé par le divinylbenzène, dont l'ouverture de maille est comprise entre environ 60 et 500, dérivé sur les phényles du polystyrène par des groupements de formule

$-(CH_2)_4CO_2H$ et
$-(CH_2)_4(CO)(NH)(CH_2)_6NH(PO_2)O$-(Oligo).

5. Procédé selon les revendications 3 ou 4 dans lequel la première et la deuxième sonde comprennent 25 à 50 bases et dans lequel la deuxième sonde est marquée par radioactivité.

6. Procédé selon la revendication 5 dans lequel le marqueur radioactif de la deuxième sonde est du $P^{32}$ appartenant à un groupement phosphate lié au carbone 5' du nucléoside 5'.

**7.** Procédé selon la revendication 6 dans lequel la solution de préhybridation et la solution d'hybridation comprennent entre environ 8 (p/v) à environ 12 % (p/v) de sulfate de dextran.

**8.** Procédé selon la revendication 7, dans lequel, avant l'étape (2) selon la revendication (1), l'acide nucléique de l'échantillon analysé est incubé en conditions d'hybridation dans une première solution d'hybridation, qui contient un excès molaire, par rapport à la concentration de l'analyte dans la solution d'hybridation, de la deuxième sonde, jusqu'à ce qu'une fraction importante de l'analyte présent se soit hybridée avec la deuxième sonde, l'étape (2) selon la revendication 1 étant ensuite initiée par combinaison avec la dite première solution d'hybridation du support solide préhybridé selon l'étape (1) de la revendication 1 et immergé dans une seconde solution d'hybridation.

**9.** Procédé selon l'une quelconque des revendications 5 à 8 dans lequel l'analyte est le génome d'un virus HIV-1, ou un fragment de celui-ci comprenant des segments comportant des séquences complémentaires de la première sonde et de la deuxième sonde.

**10.** Support solide destiné à être utilisé dans le procédé de l'une quelconque des revendications 1 à 9 avec une sonde d'oligonucléotide simple brin de 15 à 100 bases fixée sur celui-ci, et choisi parmi :

(A) un verte de silicate poreux dérivé sur les silicones par

(1) des groupements de formule

$-(CH_2)_n(NH)(CO)(CH_2)_cR_1$ et

$-(CH_2)_n(NH)(PO_2)O$-(Oligo),

avec substantiellement aucun silicone dérivé par un groupement se terminant par un groupement amine, dans lesquels c est compris entre 0 et 5, n est compris entre 2 et 8, $R_1$ est un méthyle ou un benzyle, -O-(Oligo) est la sonde d'oligonucléotide, et l'atome d'oxygène fixé sur (Oligo) est l'oxygène 5' du nucléoside 5' ou l'oxygène 3' du nucléoside 3' de la sonde ; ou

(2) des groupements de formule

$-L_1-CO_2H$ et

(i) $-L_1(CO)(NH)(CH_2)_pNH(PO_2)O$-(Oligo), ou

(ii) $-L_1(CO)(NH)(CH_2)_pSS(CH_2)_rNH(PO_2)O$-(Oligo),

dans lesquels $-L_1-$ est $-(CH_2)_n(NH)(CO)(CH_2)_m-$, dans lequel le groupement $-(CH_2)_n$ est fixé sur un atome de silicone ; et dans lesquels m, n, p et r sont égaux ou différents et sont chacun compris entre 2 et 8 ; ou

(3) des groupements de formule

$$\begin{array}{c} \phantom{-(CH_2)_n(NH)(CO)(}NH(CO)CH_3 \\ -(CH_2)_n(NH)(CO)(CH)_pSS(CH_2)_rNH(PO_2)O\text{-(Oligo)} \end{array}$$

et

$$\begin{array}{c} \phantom{-(CH_2)_n(NH)(CO)(}NH(CO)CH_3 \\ -(CH_2)_n(NH)(CO)(CH)(CH_2)SH\ ; \end{array}$$

ou

(B) un matériau macroporeux de dextran réticulé dérivé sur les oxygènes des hydroxyles, eux-mêmes portés par les carbones des fragments sucres comportant au moins un carbone avec un hydroxyle non dérivé à proximité, avec

(1) des groupements de formule

$-C(=NH)NH(CH_2)q(NE)(CO)(CH_2)_cR_1$ et
$-C(=NH)NH(CH_2)_q(NH)(CO)(CH_2)_pNH(PO_2)O$-(Oligo)

avec substantiellement aucun oxygène d'hydroxyle dérivé par un groupement se terminant par un groupement amine, ou
(2) un groupement de formule

$-C(=NH)NH(CH_2)_p(NH)(PO_2)O$-(Oligo), ou

(3) des groupements de formule

$-C(=NH)NH(CH_2)_qCO_2H$ et

(i) $L_2-NH(PO_2)O$-(Oligo), ou

(ii) $L_2-SS(CH_2)_rNH(PO_2)O$-(Oligo) dans lesquels c, q, p et r sont égaux ou différents et q est compris entre 2 et 10 et dans lesquels $L_2$ est

$-C(=NH)NH(CH_2)_q(CO)(NH)(CH_2)p$-, dans lequel le groupement -C(=NH) est lié à l'oxygène d'un hydroxyle du dextran ; ou
(4) des groupements de formule

$-C(=NH)NH(CH_2)_nSH$ et
$-C(=NH)NH(CH_2)_nSS(CH_2)_pNH(PO_2)O$-(Oligo) ; et

(C) du polystyrène éventuellement réticulé par un agent réticulant tel que le divinylbenzène, et qui est dérivé sur les groupements phényles par
(1) des groupements de formule

$-(CH_2)_sCO_2H$ et

(i) $L_3-NH(PO_2)O$-(Oligo), Ou

(ii) $L_3-SS(CH_2)_nNH(PO_2)O$-(Oligo), dans lesquels $L_3$ est $-(CH_2)_s(CO)(NH)(CH_2)_p$-, dans lequel le groupement $(CH_2)_s$ est lié à un groupement phényle du polystyrène et dans lequel s, p et n sont égaux ou différents et s est compris entre 2 et 10 ; ou
(2) des groupements de formule

$-(CH_2)_s(CO)(NH)(CH_2)_p(NH)(CO)(CH_2)_cR_1$ et
$-(CH_2)_s(CO)(NH)(CH_2)_p(NH)(PO_2)O$-(Oligo),

avec substantiellement aucun groupement phényle dérivé par un groupement terminé par un groupement amine; ou
(3) des groupements de formule

$(CH_2)_s(CO)(NH)(CH_2)_pSH$, et
$(CH_2)_s(CO)(NH)(CH_2)_pSS(CH_2)_nNH(PO_2)O$-(Oligo).

**11.** Support solide selon la revendication 10 sur lequel l'oligonucléotide est fixé par l'oxygène 5' du nucléoside 5' et pour lequel le support est choisi parmi :
(A) un verte à pore contrôlé, sous forme de billes de diamètre compris entre environ 100 et 200 $\mu$m (microns) et dont le diamètre des pores est compris entre environ 0,04 $\mu$m et 0,06 $\mu$m (400 à 600 Angstroms) et dérivé sur les silicones par des groupements de formule

$-(CH_2)_a(NH)(CO)(CH_2)_bCH_3$ et
$-(CH_2)_a(NH)(PO_2)O$-(Oligo)

avec substantiellement aucun silicone dérivé par un groupement terminé par un groupement amine, dans lesquels a est égal à 3 ou 6 et b est égal à 0, 1, ou 2 ; et

(B) un matériau macroporeux, obtenu par réticulation d'allyldextran par du N,N'-méthylènebisacrylamide, sous forme de billes dont le diamètre, à l'état humide, est compris entre environ 30 et 120 µm (microns) et le volume d'exclusion pour les dextrans est compris entre environ $10^6$ et $10^8$ daltons, et dérivé, sur les oxygènes des hydroxyles, eux-mêmes sur des carbones des fragments sucres comportant au moins un carbone portant un hydroxyle non dérivé à proximité, par des groupements de formule

$-C(=NH)NH(CH_2)_6NH(CO)(CH_2)_bCH_3$ et
$-C(=NH)NH(CH_2)_6NH(CO)(CH_2)_6NH(PO_2)O\text{-(Oligo)}$,

avec substantiellement aucun oxygène d'hydroxyle dérivé par un groupement terminé par un groupement amine, ou un groupement de formule

$-C(=NH)NH(CH_2)_6NH(PO_2)O\text{-(Oligo)}$.

**12.** Support solide selon la revendication 10 pour lequel l'oligonucléotide est fixé sur le support solide par l'oxygène 5' du nucléoside 5' et pour lequel le support est choisi parmi :

(A) un verre à pores contrôlés, sous forme de billes de diamètre compris entre environ 100 et 200 µm (microns) et dont le diamètre des pores est compris entre environ 0,04 µm et 0,06 µm (400 à 600 Angstroms) et dérivé sur les silicones par des groupements de formule

$-(CH_2)_3(NH)(CO)(CH_2)_2CO_2H$ et
$-(CH_2)_3NH(CO)(CH_2)_2(CO)(NH)(CH_2)_6NH(PO_2)O\text{-(Oligo)}$

et

(B) un matériau macroporeux, obtenu par réticulation d'allyldextran par du N,N'-méthylènebisacrylamide, sous forme de billes dont le diamètre, à l'état humide, est compris entre environ 30 et 120 µm (microns) et le volume d'exclusion pour les dextrans est compris entre environ $10^6$ et $10^8$ daltons, et dérivé, sur les oxygènes des hydroxyles, eux-mêmes sur des carbones des fragments sucres comportant au moins un carbone portant un hydroxyle non dérivé à proximité, par des groupements de formule

$-C(=NH)NH(CH_2)_5CO_2H$ et
$-C(=NH)NH(CH_2)_5(CO)(NH)(CH_2)_6NH(PO_2)O\text{-(Oligo)}$,

et

(C) 0 à environ 16 % de billes de polystyrène réticulé par le divinylbenzène, dont l'ouverture de maille est comprise entre environ 60 et 500, dérivé sur les phényles du polystyrène par des groupements de formule

$-(CH_2)_4CO_2H$ et
$-(CH_2)_4(CO)(NH)(CH_2)_6NH(PO_2)O\text{-(Oligo)}$.

**13.** Support solide selon les revendications 11 ou 12 pour lequel l'oligonucléotide comprend 25 à 50 bases.

**14.** Support solide selon la revendication 13 pour lequel l'oligonucléotide est un ADN ou un ARN comportant une séquence complémentaire de la séquence d'un segment du génome de HIV-1.

**15.** Kit de test pour mettre en oeuvre le procédé de l'une quelconque des revendications 1 à 9 pour la détection, à l'aide d'une analyse en sandwich avec sonde d'acide nucléique, d'un analyte d'acide nucléique simple brin, ce kit comprenant (A) un support solide selon l'une quelconque des revendications 12 à 16, sur lequel (Oligo) possède une séquence complémentaire de la séquence d'un premier segment du dit analyte et (B) une deuxième sonde, qui consiste en un oligonucléotide simple brin possédant une séquence complémentaire de la séquence d'un deuxième segment du dit analyte et qui est marquée pour pouvoir être détectée, étant entendu que la séquence de la deuxième sonde n'est

pas complémentaire de celle de la première sonde et que le deuxième segment ne recouvre pas le premier segment.

16. ARN ou ADN à utiliser dans les procédés de l'une quelconque des revendications 1 à 9 avec une séquence choisie parmi l'ensemble comprenant :

5'-CAAAAACTATTCTTAAACCTACCAAGCCTC-3'
5'-TATTACATTTTAGAATCGCAAAACCAGCC-3'
5'-TAGGTTTCCCTGAAACATACATATGGTGT-3'

5'-TGGTCTGCTAGTTCAGGGTCTACTTGTGTGC-3'
5'-CACCTAGGGCTAACTATGTGTCCTAATAAGG-3'
5'-TTTCGTAACACTAGGCAAAGGTGGCTTTATC-3'
5'-TGGTCTTCTGGGGCTTGTTCCATCTATCCTC-3'
5'-AGGGAAAATGTCTAACAGCTTCATTCTTAAC-3'
5'-AAATGGATAAACAGCAGTTGTTGCAGAATTC-3'
5'-TCGAGTAACGCCTATTCTGCTATGTCGACAC-3'
5'-CTGTGTAATGACTGAGGTGTTACAACTTGT-3'
5'-TCTAATTACTACCTCTTCTTCTGCTAGACT-3'
5'-AATATGTTGTTATTACCAATCTAGCAT-3', et

toute séquence complémentaire de l'une quelconque de celles spécifiées ci-dessus, étant entendu que, dans un ARN, le T des séquences spécifiées ci-dessus représente une uridine.